# EUROPEAN PATENT SPECIFICATION

(11) **EP 2 418 216 B1**
(45) Date of publication and mention of the grant of the patent: **28.10.2015**
(21) Application number: 10808203.3
(22) Date of filing: 09.08.2010
(51) Int. Cl.: C07K 14/47, A61K 51/00, G01T 1/161

(54) **MOLECULAR PROBE FOR IMAGING OF PANCREATIC ISLET AND PRECURSOR THEREOF, AND USE OF THE MOLECULAR PROBE OR THE PRECURSOR**
MOLEKULARE SONDE ZUR BILDGEBUNG DER LANGERHANS-INSELN UND VORLÄUFER DAVON SOWIE VERWENDUNG DER MOLEKULAREN SONDE ODER DES VORLÄUFERS
SONDE MOLÉCULAIRE POUR L'IMAGERIE D'UN ÎLOT PANCRÉATIQUE ET PRÉCURSEUR CORRESPONDANT, UTILISATION DE LA SONDE MOLÉCULAIRE OU DU PRÉCURSEUR

(30) Priority: 10.08.2009 JP 2009185470; 10.08.2009 US 232619 P; 30.11.2009 JP 2009272445; 24.03.2010 JP 2010068257; 25.03.2010 US 282741 P
(43) Date of publication of application: 15.02.2012
(73) Proprietor: Kyoto University, Kyoto-shi, Kyoto 606-8501 (JP); ARKRAY, Inc., Minami-ku Kyoto-shi Kyoto 601-8045 (JP)
(72) Inventor: INAGAKI, Nobuya, Kyoto-shi Kyoto 606-8501 (JP); SAJI, Hideo, Kyoto-shi Kyoto 606-8501 (JP); TOYODA, Kentaro, Kyoto-shi Kyoto 606-8501 (JP); KIMURA, Hiroyuki, Kyoto-shi Kyoto 606-8501 (JP); OGAWA, Yu, Kyoto-shi Kyoto 606-8501 (JP); HIRAO, Konomu, Kyoto-shi Kyoto 601-8045 (JP); NAGAKAWA, Kenji, Kyoto-shi Kyoto 601-8045 (JP); MATSUDA, Hirokazu, Kyoto-shi Kyoto 601-8045 (JP)
(74) Representative: Jones, Elizabeth Louise
(86) International application number: PCT/JP2010/063489
(87) International publication number: WO 2011/019020

(56) References cited:
- US-B1- 6 858 576
- BEHE, M. ET AL.: "Are radiolabeled GLP-1 receptor antagonists useful for scintigraphy?", J. NUCL. MED. MEETING ABSTRACTS, vol. 50, no. SUP.2, May 2009 (2009-05), page 327, XP002683016,
- GÖKE R ET AL: "Exendin-4 is a high potency agonist and truncated exendin-(9-39)-amide an antagonist at the glucagon-like peptide 1-(7-36)-amide receptor of insulin-secreting beta-cells", JOURNAL OF BIOLOGICAL CHEMISTRY, AMERICAN SOCIETY FOR BIOCHEMISTRY AND MOLECULAR BIOLOGY, US, vol. 268, no. 26, 15 September 1993 (1993-09-15), pages 19650-19655, XP002966930, ISSN: 0021-9258
- SCHIRRA J ET AL: "Exendin(9-39)amide is an antagonist of glucagon-like peptide-1(7-36)amide in humans", JOURNAL OF CLINICAL INVESTIGATION, AMERICAN SOCIETY FOR CLINICAL INVESTIGATION, US, vol. 101, no. 7, 1 April 1998 (1998-04-01) , pages 1421-1430, XP002306100, ISSN: 0021-9738, DOI: 10.1172/JCI1349
- B D GREEN: "Chronic treatment with exendin(9-39)amide indicates a minor role for endogenous glucagon-like peptide-1 in metabolic abnormalities of obesity-related diabetes in ob/ob mice", JOURNAL OF ENDOCRINOLOGY, vol. 185, no. 2, 1 May 2005 (2005-05-01), pages 307-317, XP55037142, ISSN: 0022-0795, DOI: 10.1677/joe.1.05876
- KIMURA, H. ET AL.: "Development of in vivo imaging agents targeting glucagon-like peptide- 1 receptor (GLP-1R) in pancreatic islets.", J. NUCL. MED. MEETING ABSTRACTS, vol. 50, no. SUP.2, May 2009 (2009-05), page 326, XP002683017,
- DAMIAN WILD ET AL: "[Lys40(Ahx-DTPA-111In)NH2]exendin-4, a very promising ligand for glucagon-like peptide-1 (GLP-1) receptor targeting.", THE JOURNAL OF NUCLEAR MEDICINE, vol. 47, no. 12, 1 December 2006 (2006-12-01), pages 2025-2033, XP55037206, ISSN: 0161-5505
- WICKI ANDREAS ET AL: "[Lys40(Ahx-DTPA-111In)NH2]-Exendin-4 is a highly efficient radiotherapeutic for glucagon-like peptide-1 receptor-targeted therapy for insulinoma", CLINICAL CANCER RESEARCH, THE AMERICAN ASSOCIATION FOR CANCER RESEARCH, US, vol. 13, no. 12, 15 June 2007 (2007-06-15) , pages 3696-3705, XP002561708, ISSN: 1078-0432, DOI: 10.1158/1078-0432.CCR-06-2965
- MAARTEN BROM ET AL: "Radiolabelled GLP-1 analogues for in vivo targeting of insulinomas", CONTRAST MEDIA & MOLECULAR IMAGING, vol. 7, no. 2, 1 March 2012 (2012-03-01), pages 160-166, XP55037204, ISSN: 1555-4309, DOI: 10.1002/cmmi.475
- BEATRICE WASER ET AL: "Value of the radiolabelled GLP-1 receptor antagonist exendin(9â 39) for targeting of GLP-1 receptor-expressing pancreatic tissues in mice and humans", EUROPEAN JOURNAL OF NUCLEAR MEDICINE AND MOLECULAR IMAGING, SPRINGER, BERLIN, DE, vol. 38, no. 6, 6 January 2011 (2011-01-06), pages 1054-1058, XP019902585, ISSN: 1619-7089, DOI: 10.1007/S00259-010-1701-0
- BEHE, M. ET AL.: 'Are radiolabeled GLP-1 receptor antagonists useful for scintigraphy?' J. NUCL. MED. MEETING ABSTRACTS vol. 50, no. SUP.2, May 2009, page 327
- GOKE, R. ET AL.: 'Exendin-4 Is a High Potency Agonist and Truncated Exendin-(9-39)-amide an Antagonist at the Glucagon-like Peptide 1-(7- 36)-amide Receptor of Insulin-secreting beta- Cells.' J. BIOL. CHEM. vol. 268, no. 26, 1993, pages 19650 - 19655
- SCHIRRA, J. ET AL.: 'Exendin(9-39)amide Is an Antagonist of Glucagon-like Peptide-1(7-36) amide in Humans.' J. CLIN. INVEST. vol. 101, no. 7, 1998, pages 1421 - 1430
- GREEN, B. D. ET AL.: 'Chronic treatment with exendin(9-39)amide indicates a minor role for endogenous glucagon-like peptide-1 in metabolic abnormalities of obesity-related diabetes in ob/ob mice.' J. ENDOCRINOL. vol. 185, 2005, pages 307 - 317
- KIMURA, H. ET AL.: 'Development of in vivo imaging agents targeting glucagon-like peptide- 1 receptor (GLP-1R) in pancreatic islets.' J. NUCL. MED. MEETING ABSTRACTS vol. 50, no. SUP.2, May 2009, page 326

## Description

### Technical Field

The present invention relates to a molecular probe for imaging of pancreatic islets and a precursor thereof, and the use of the molecular probe and the precursor.

### Background Art

Today, type-II diabetics are continuously increasing in Japan, and the estimated number of the same exceeds 8,200,000. As a measure against this increase, interventions for preventing diabetes from developing have been made based on the glucose tolerance test, resulting, however, in unsatisfactory effects. The cause is as follows: at such a borderline stage that functional abnormalities are found by the glucose tolerance test, disorders of pancreatic islets have already advanced to a high degree, and this stage possibly is too late as a time for starting interventions.

More specifically, in the diabetes developing process, the amount of pancreatic islets (particularly, the amount of pancreatic β-cells) decreases prior to the occurrence of glucose tolerance abnormalities. Therefore, when functional abnormalities are detected or there are subjective symptoms, diabetes has already reached the stage where it is too difficult to be treated. On the other hand, if a decrease in the amount of pancreatic islets and/or the amount of pancreatic β-cells can be detected at an early stage, there is a possibility for the prevention and treatment of diabetes. Therefore, a noninvasive imaging technique of pancreatic islets, particularly a noninvasive imaging technique of pancreatic islets for determining the amount of the pancreatic islets and/or the amount of pancreatic β-cells, has been desired for the prevention and diagnosis of diabetes. Among these, a molecular probe that enables pancreatic islets imaging, preferably pancreatic β-cell imaging or measurement of the amount of pancreatic β-cells, has been desired in particular.

In designing a molecular probe for imaging of pancreatic islets, various target molecules in pancreatic cells, particularly functional proteins specific in the β-cells, are being researched. Among these, GLP-1R (glucagon-like peptide-1 receptor) is being researched as a target molecule; GLP-1R is distributed in pancreatic β-cells, and is a seven-transmembrane G protein coupled receptor. As a molecular probe for imaging pancreatic islet β-cells, for example, a derivative of exendin-4(9-39) as a GLP-1R antagonist has been researched (e.g., Non-Patent Document 1).

As an alternative probe for GLP-1R imaging, for the purpose of imaging a GLP-1R positive tumor, a derivative of exendin-4 as a GLP-1R agonist or a derivative of exendin-4(9-39) as a GLP-1R antagonist has been researched (e.g., Non-Patent Document 2). Non-Patent Documents 3 and 4 disclose the use of Lys⁴⁰(Ahx-DTPA-¹¹¹In)NH₂]exendin-4 as a ligand for GLP-1R targeting.

However, a novel molecular probe for imaging of pancreatic islets that enables noninvasive three-dimensional imaging of pancreatic islets is ultimately preferred.

### Prior Art Document

### Non-Patent Documents

[Non-Patent Document 1] H. Kimura et al. Development of in vivo imaging agents targeting glucagons-like peptide-1 receptor (GLP-1R) in pancreatic islets, 2009 SNM Annual Meeting, abstract, Oral Presentations No.326
[Non-Patent Document 2] M. Behe et al. Are radiolabeled GLP-1 receptor antagonists useful for scintigraphy? 2009 SNM Annual Meeting, abstract, Oral Presentations No.327 and Behe et al. 2009, J. Nucl. Med Meeting Abstracts, Vol. 50, No. Suppl. 2, p327
[Non-Patent Document 3] Wild et al. 2006, J. Nuclear Med., Vol. 47(12), p2025-2033
[Non-Patent Document 4] Andreas et al. 2007, Clin. Cancer Res., Am. Assoc. Cancer Res., US, Vol. 13(12), p3696-3705

### Disclosure of Invention

### Problem to be Solved by the Invention

The present invention provides a molecular probe for imaging of pancreatic islets that enables noninvasive three-dimensional imaging of pancreatic islets.

### Means for solving problem

The present invention provides use of a precursor of a molecular probe (hereinafter referred to as a molecular probe precursor) for imaging of pancreatic islets, the precursor comprising any one of the following polypeptides:
a polypeptide represented by any one of the following formulae (1) to (4),
   *-DLSK* QMEEEAVRLFIEWLK* NGGPSSGAPPPSK-NH₂ (1) (SEQ ID NO. 1)
   *-LSK*QMEEEAVRLFIEWLK* NGGPSSGAPPPSK-NH₂ (2) (SEQ ID NO. 2)
   *-SK*QMEEEAVRLFIEWLK* NGGPSSGAPPPSK-NH₂ (3) (SEQ ID NO. 3)
   *-K*QMEEEAVRLFIEWLK* NGGPSSGAPPPSK-NH₂ (4) (SEQ ID NO. 4),
in the formulae (1) to (4), ^{*}- indicates that the α-amino group at the N-terminus is protected by a protecting group or modified with a modifying group having no electric charge; K* indicates that the amino group of the side chain of the lysine is protected by a protecting group; and -NH₂ indicates that the carboxyl group at the C-terminus is amidated, wherein said precursor is used to obtain the molecular probe and said molecular probe binds to pancreatic islets, wherein the amino group of the side chain of the lysine at the C-terminus is labeled with a labeling compound comprising an aromatic ring having a radioactive nuclide.

As an alternative embodiment, the present invention provides a molecular probe for imaging of pancreatic islets (or use of the molecular probe for imaging), the molecular probe comprising any one of the following polypeptides:
a polypeptide represented by any one of the following formulae (5) to (8),
   Z-DLSKQMEEEAVRLFIEWLKNGGPSSGAPPPSX-NH₂ (5) (SEQ ID NO. 5)
   Z-LSKQMEEEAVRLFIEWLKNGGPSSGAPPPSX-NH₂ (6) (SEQ ID NO. 6)
   Z-SKQMEEEAVRLFIEWLKNGGPSSGAPPPSX-NH₂ (7) (SEQ ID NO. 7)
   Z-KQMEEEAVRLFIEWLKNGGPSSGAPPPSX-NH₂ (8) (SEQ ID NO. 8)
in the formulae (5) to (8), X represents a lysine residue having the amino group of the side chain labeled with a radioactive nuclide, where said amino group of the side chain of the lysine is bonded to a group comprising an aromatic ring represented by chemical formula (III) below,

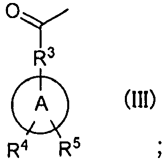

Z- indicates that an α-amino group at the N-terminus is unmodified, or modified with a modifying group having no electric charge; and, -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group.

### Effects of the Invention

The present invention enables imaging of pancreatic islets, preferably three-dimensional imaging of pancreatic islets, and more preferably noninvasive imaging of pancreatic islets by, for example, positron emission tomography (PET), single photon emission computed tomography (SPECT) and the like.

### Brief Description of Drawings

[FIG. 1] FIGS. 1A and 1B are graphs showing exemplary result of variations with time of biodistribution of a molecular probe as described herein.
[FIG. 2] FIGS. 2A and 2B are graphs showing exemplary result of variations with time of biodistribution of a molecular probe of Reference Example 1.
[FIG. 3] FIGS. 3A and 3B are graphs showing exemplary result of variations with time of biodistribution of a molecular probe of Reference Example 2.
[FIG. 4] FIG. 4 is a graph showing an exemplary result of a blocking experiment using a molecular probe of Example 1.
[FIG. 5] FIG. 5 is an image showing an exemplary result of an imaging analysis on pancreas section using the molecular probe of Example 1.
[FIG. 6] FIG. 6A and 6B are PET images showing an exemplary result of imaging of pancreatic islets using the molecular probe of Example 1.
[FIG. 7] FIGS. 7A and 7B are graphs other showing exemplary result of variations with time of biodistribution of a molecular probe as described herein.
[FIG. 8] FIG8 is a graph showing an exemplary result of a binding assay in Example.
[FIG. 9] FIGS. 9A and 9B are graphs showing another exemplary result of variations with time of biodistribution of a molecular probe as described herein.
[FIG. 10] FIG. 10 is an image showing exemplary result of an imaging analysis on pancreas section using a molecular probe of Example 3.
[FIG. 11] FIGS 11A to 11C show exemplary SPECT images obtained by using a molecular probe of Example 4.

### Description of the Invention

The diameter of a pancreatic islet is, for example, approximately 50 to 500 µm in the case of humans. In order to noninvasively image or quantitate such pancreatic islets in a living body, a molecular probe, for example, is considered to be necessary that can show specific accumulation in pancreatic islets, thereby making contrast between pancreatic islets and surrounding organs. Research and development of molecular probes has therefore been conducted.

For example, Non-Patent Document 2 reports the research on the affinity of Lys⁴⁰(Ahx-DTPA-¹¹¹In)Exendin-4(9-39), which is a derivative of exendin-4(9-39), with respect to GLP-1R in GLP-1R-positive tumor cells and pancreatic islet cells. According to this document, the following was proved consequently: the uptake of Lys⁴⁰(Ahx-DTPA-¹¹¹In)Exendin-4(9-39) in pancreatic islets was about 0.4%, the uptake thereof in the GLP-1R-positive tumor cells was about 7.5%. In other words, the results show that Lys⁴⁰(Ahx-DTPA-¹¹¹In)Exendin-4(9-39) has a low affinity with respect to GLP-1R. Therefore, for example, under the present circumstances, a novel molecular probe that can show specific uptake in the pancreatic islets and produce a contrast with the surrounding organs is desired.

The present invention is based on a finding that a molecular probe obtained by labeling and deprotecting the precursor for molecular probe for imaging and also the molecular probe for imaging enable for example noninvasive three-dimensional imaging of pancreatic islets by means of PET, SPECT or the like, and ensure quantitativeness. In other words, the present invention preferably achieves an effect of enabling noninvasive three-dimensional imaging of pancreatic islets. Further, since the present invention provides a molecular probe that enables more specific accumulation in the pancreatic islets in comparison with the molecular probes as described in the Non-Patent Documents 1 and 2, the present invention preferably achieves an effect of enabling imaging of pancreatic islets for quantitation.

As described above, it is known that in the diabetes developing process, the amount of pancreatic islets decreases prior to the occurrence of glucose tolerance abnormalities. Therefore, by performing imaging of pancreatic islets and/or determining the amount of pancreatic islets, for example, minute changes in pancreatic islets can be found in a state prior to the development of diabetes or in an initial stage of the same, whereby the ultra-early detection and diagnosis of diabetes are enabled. Thus, the molecular probe precursor for imaging of the present invention is useful for the prevention, early detection, and diagnosis of diabetes, preferably for the ultra-early detection and diagnosis of diabetes.

More specifically, the present invention relates to the following contents.
[1] Use of a precursor of a molecular probe for imaging of pancreatic islets, the precursor comprising any one of the following polypeptides: a polypeptide represented by any one of the following formulae (1) to (4),
   *-DLSK* QMEEEAVRLFIEWLK* NGGPSSGAPPPSK-NH₂ (1) (SEQ ID NO. 1)
   *-LSK* QMEEEAVRLFIEWLK* NGGPSSGAPPPSK-NH₂ (2) (SEQ ID NO. 2)
   *-SK* QMEEEAVRLFIEWLK* NGGPSSGAPPPSK-NH₂ (3) (SEQ ID NO. 3)
   *-K* QMEEEAVRLFIEWLK* NGGPSSGAPPPSK-NH₂ (4) (SEQ ID NO. 4)
   in the formulae (1) to (4), *- indicates that the α-amino group at the N-terminus is protected by a protecting group or modified with a modifying group having no electric charge, and K* indicates that the amino group of the side chain of the lysine is protected by a protecting group, and -NH₂ indicates that the carboxyl group at the C-terminus is amidated, wherein said precursor is used to obtain the molecular probe and said molecular probe binds to pancreatic islets, wherein the amino group of the side chain of a lysine at the C-terminus is labeled with a labeling compound comprising an aromatic ring having a radioactive nuclide;
[2] The use of a precursor of a molecular probe for imaging of pancreatic islets according to [1], wherein the modifying group having no electric charge is selected from the group consisting of acetyl group, benzyl group, benzyloxymethyl group, o-bromobenzyloxycarbonyl group, t-butyl group, t-butyldimethylsilyl group, 2-chlorobenzyl group, 2,6-dichlorobenzyl group, cyclohexyl group, cyclopentyl group, isopropyl group, pivalyl group, tetrahydropyran-2-yl group, tosyl group, trimethylsilyl group, and trityl group;
[3] A method for producing a molecular probe for imaging of pancreatic islets, comprising labeling and deprotecting the precursor of the molecular probe for imaging of pancreatic islets as defined in [1] or [2], wherein the labeling of the precursor of the molecular probe comprises labeling of the amino group of the side chain of the lysine at the C-terminus with a labeling compound comprising an aromatic ring having a radioactive nuclide;
[4] The method for producing a molecular probe for imaging of pancreatic islets according to [3], wherein the labeling compound comprising the aromatic ring comprises a group represented by chemical formula (I) below, 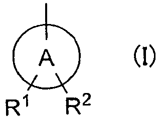 wherein A represents any of an aromatic hydrocarbon group and an aromatic heterocycle; R¹ represents a substituent comprising ¹⁸F, ⁷⁵Br, ⁷⁶Br, ⁷⁷Br, ¹²³I, ¹²⁴I, ¹²⁵I, or ¹³¹I; and R² represents a hydrogen atom or one or more substituents different from the substituents represented by R¹.;
[5] A molecular probe for imaging of pancreatic islets, comprising any one of the following polypeptides:
   a polypeptide represented by any one of the following formulae (5) to (8),
      Z-DLSKQMEEEAVRLFIEWLKNGGPSSGAPPPSX-NH₂ (5) (SEQ ID NO. 5)
      Z-LSKQMEEEAVRLFIEWLKNGGPSSGAPPPSX-NH₂ (6) (SEQ ID NO. 6)
      Z-SKQMEEEAVRLFIEWLKNGGPSSGAPPPSX-NH₂ (7) (SEQ ID NO. 7)
      Z-KQMEEEAVRLFIEWLKNGGPSSGAPPPSX-NH₂ (8) (SEQ ID NO. 8)
   in the formulae (5) to (8), X represents a lysine residue having the amino group of the side chain labeled with a radioactive nuclide, where said amino group of the side chain of the lysine is bonded to a group comprising an aromatic ring represented by chemical formula (III) below, 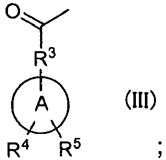 Z- indicates that the α-amino group at the N-terminus is unmodified or modified with a modifying group having no electric charge; and, -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group;
[6] Use of a molecular probe for imaging of pancreatic islets, comprising any one of the following polypeptides: a polypeptide represented by any one of the following formulae (5) to (8),
   Z-DLSKQMEEEAVRLFIEWLKNGGPSSGAPPPSX-NH₂ (5) (SEQ ID NO. 5)
   Z-LSKQMEEEAVRLFIEWLKNGGPSSGAPPPSX-NH₂ (6) (SEQ ID NO. 6)
   Z-SKQMEEEAVRLFIEWLKNGGPSSGAPPPSX-NH₂ (7) (SEQ ID NO. 7)
   Z-KQMEEEAVRLFIEWLKNGGPSSGAPPPSX-NH₂ (8) (SEQ ID NO. 8)
   in the formulae (5) to (8), X represents a lysine residue having the amino group of the side chain labeled with a radioactive nuclide, where said amino group of the side chain of the lysine is bonded to a group comprising an aromatic ring represented by chemical formula (III) below, 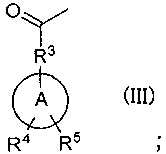 Z- indicates that an α-amino group at the N-terminus is unmodified or modified with a modifying group having no electric charge; and -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group.;
[7] A kit for preparing a molecular probe for imaging of pancreatic islets, comprising the precursor of a molecular probe for imaging of pancreatic islets as defined in [3], wherein the kit further comprising a compound for labeling the precursor of a molecular probe for imaging of pancreatic islets, wherein the compound comprises an aromatic ring having a radioactive nuclide;
[8] The kit according to [7], wherein the compound comprising the aromatic ring is a compound having a group represented by chemical formula (I) below, 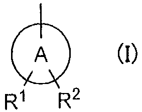 in the formula (I), A represents any of an aromatic hydrocarbon group or an aromatic heterocycle, R¹ represents a substituent comprising ¹⁸F, ⁷⁵Br, ⁷⁶Br, ⁷⁷Br, ¹²³I, ¹²⁴I, ¹²⁵I, or ¹³¹I, and R² represents a hydrogen atom or one or more substituents different from the substituents represented by R¹.;
[9] A kit for performing pancreatic islets imaging, comprising the molecular probe for imaging of pancreatic islets as defined in [5] or [6];
[10] A method for imaging of pancreatic islets comprising:
   (A) labeling and deprotecting the precursor of a molecular probe as defined in [3], wherein the labeling of the precursor comprises labeling the amino group of the side chain of the lysine at the C-terminus with a labeling compound comprising an aromatic ring having a radioactive nuclide, and imaging pancreatic islets by using the labeled and deprotected molecular probe for imaging of pancreatic islets, or
   (B) detecting a signal of the molecular probe as defined in [5] or [6] from an analyte to which the probe has been administered;
[11] A method for determining the amount of pancreatic islets, comprising:
   (A) preparing a molecular probe for imaging of pancreatic islets by labeling and deprotecting the precursor of the molecular probe as defined in [3], wherein the labeling of the precursor comprises labeling the amino group of the side chain of the lysine at the C-terminus with a labeling compound comprising an aromatic ring having a radioactive nuclide; and
      calculating the amount of pancreatic islets based on the results of imaging of pancreatic islets with use of the molecular probe, or
   (B) detecting a signal of the molecular probe as defined in [5] or [6] for imaging of pancreatic islets from an analyte to which the probe has been administered; and calculating the amount of pancreatic islets based on the detected signal of the molecular probe for imaging of pancreatic islets.
[12] The molecular probe as defined in [5] or [6] for use in prevention, treatment, or *in vivo* diagnosis of diabetes, wherein in said prevention, treatment, or diagnosis said molecular probe is used in the imaging of pancreatic islets.

### [Imaging of Pancreatic Islet]

In the present specification, the "imaging of pancreatic islet" refers to molecular imaging of pancreatic islets, and includes the imaging of *in vivo* spatial and/or time distribution of pancreatic islets. Further, in the present invention, the imaging of pancreatic islets preferably images pancreatic β-cells as target molecules, and more preferably images the GLP-1 receptor of the pancreatic β-cell as target molecules, from the viewpoint of the prevention, treatment, and diagnosis of diabetes. Still further, in the present invention, the imaging of pancreatic islets preferably is noninvasive three-dimensional imaging, from the viewpoint of quantitativeness of an amount of pancreatic islets and applicability to humans. The method of imaging is not limited particularly, if it is a method that enables noninvasive imaging of pancreatic islet. Various methods are usable such as a method that utilizes positron emission tomography (PET), a method that utilizes single photon emission computed tomography (SPECT), a method that utilizes magnetic resonance imaging (MRI), and methods that utilize X-rays, visible light, fluorescence, near infrared light, ultrasonic waves and the like. Among these, PET and SPECT are preferred, from the viewpoint of the quantitation of an amount of pancreatic islets with use of the molecular probe precursor of the present invention.

### [Molecular Probe Precursor of the Present Invention]

A molecular probe precursor for use according to the present invention is a molecular probe precursor for imaging of pancreatic islets, the precursor containing any one of the following polypeptides: a polypeptide represented by any one of the formulae (1) to (4), the polypeptide being capable of binding to pancreatic islets after being labeled and deprotected, wherein the molecular probe is for use in imaging of pancreatic islet. Preferably, a molecular probe precursor for use according to the present invention is a molecular probe precursor for imaging of pancreatic islets, the precursor consisting of any one of the following polypeptides: a polypeptide represented by any one of the formulae (1) to (4), the polypeptide being capable of binding to pancreatic islets after being labeled and deprotected, wherein the molecular probe is for use in imaging of pancreatic islet.

The molecular probe precursor for use according to the present invention is a polypeptide for use in imaging of pancreatic islets, and a polypeptide is represented by any one of the above-mentioned formulae (1) to (4). Amino acid sequences of polypeptides of the foregoing formulae (1) to (4) are the amino acid sequences according to SEQ ID NOS. 1 to 4 shown in the Sequence Listing, respectively. It should be noted that protecting groups for protecting amino groups are bonded to the following amino groups: amino groups of side chains of the lysines at positions 4 and 19 of the polypeptide of the foregoing formula (1), amino groups of side chains of the lysines at positions 3 and 18 of the polypeptide of the foregoing formula (2), amino groups of side chains of the lysines at positions 2 and 17 of the polypeptide of the foregoing formula (3), and amino groups of side chains of the lysines at positions 1 and 16 of the polypeptide of the foregoing formula (4). Further, the α-amino group at the N-terminus of each of the polypeptides of the foregoing formulae (1) to (4) is bonded to a protecting group for protecting the amino group, or the α-amino group is modified with a modifying group having no electric charge. The carboxyl group at the C-terminus of each of the polypeptides of the foregoing formulae (1) to (4) is amidated by an amino group, from a viewpoint of improving the property of binding to the pancreatic β-cells.

When the molecular probe precursors for use according to the present invention that contain the polypeptides of the foregoing formulae (1) to (4) are labeled with a labeling system for labeling an amino group that will be described later, an amino group of the side chain of the lysine at the C-terminus not protected by a protecting group can be labeled. Namely, the amino group of the side chain of the lysine at position 32 of the polypeptide of the foregoing formula (1), the amino group of the side chain of the lysine at position 31 of the polypeptide of the foregoing formula (2), the amino group of the side chain of the lysine at position 30 of the polypeptide of the foregoing formula (3), and the amino group of the side chain of the lysine at position 29 of the polypeptide of the foregoing formula (4), will be labeled.

Here, the sequence of the amino acids at positions 1 to 31 of the foregoing formula (1) (SEQ ID NO. 1 in the Sequence Listing) coincide with the amino acid sequence of exendin(9-39) except for a protecting group for bonding to the amino group of the side chain of a lysine and a protecting group or modifying group for bonding to the α-amino group at the N-terminus. It is known that exendin(9-39) bonds to GLP-1R (glucagon-like peptide-1 receptor) expressed on the pancreatic β-cell. The molecular probe obtained by labeling and deprotecting the molecular probe precursor for use according to the present invention (this molecular probe is hereinafter referred to also as "molecular probe of the present invention") also is capable of binding to pancreatic islets, and preferably the pancreatic β-cells.

In the present specification, the description of "being capable of binding to pancreatic islets" herein means the following: from the viewpoint of applying the present invention to the quantitation of the pancreatic islets and a use of the examination and diagnosis, the molecular probe as described herein preferably is capable of binding to the pancreatic β-cells, more preferably is at least specific to the pancreatic β-cells in the pancreas, and further more preferably is at least specific to such an extent that a signal thereof does not overlap a signal of another organ/tissue during a signal detection in noninvasive imaging with respect to humans.

It should be noted that the molecular probe precursor as described herein can be produced by peptide synthesis in accordance with a typical method such as an Fmoc method, and the peptide synthesis method is not limited particularly.

The molecular probe precursor for use according to the present invention, as described above, can be used in imaging of pancreatic islets, and preferably it is used in noninvasive imaging of pancreatic islets from the viewpoint of the application of the same to the examination and diagnosis for humans, and preferably is used in imaging of pancreatic islets for quantitating the amount of the pancreatic islets from the same viewpoint. Further, the molecular probe precursor for use according to the present invention preferably is used in imaging of pancreatic islets for the prevention and treatment for diabetes or for the diagnosis of diabetes. Such imaging of pancreatic islets may be performed by PET or SPECT, for example.

### [Protecting Group]

The protecting group for the molecular probe precursor for use according to the present invention is intended to protect the other amino group than a specific amino group of the molecular probe as described herein while the specific amino group is being labeled, in which the specific amino group is the amino group of the side chain of the lysine positioned on the C-terminus side in the molecular probe precursor for use according to the present invention. As the protecting group, any known protecting group capable of performing such a function can be used. The protecting group is not limited particularly, and examples of the same include 9-fluorenyl methyloxycarbonyl group (Fmoc), tert-butoxycarbonyl group (Boc), benzyloxycarbonyl group (Cbz), 2,2,2-trichloroethoxycarbonyl group (Troc), allyloxycarbonyl group (Alloc), amino group, alkyl groups having 3 to 20 carbon atoms, 9-fluoreneacetyl group, 1-fluorenecarboxylic acid group, 9-fluorenecarboxylic acid group, 9-fluorenone-1-carboxylic acid group, benzyloxycarbonyl group, xanthyl group (Xan), trityl group (Trt), 4-methyltrityl group (Mtt), 4-methoxytrityl group (Mmt), 4-methoxy2,3,6-trimethyl-benzenesulfonyl group (Mtr), mesitylene-2-sulfonyl group (Mts), 4,4-dimethoxybenzohydryl group (Mbh), tosyl group (Tos), 2,2,5,7,8-pentamethylchroman-6-sulfonyl group (Pmc), 4-methylbenzyl group (MeBzl), 4-methoxybenzyl group (MeOBzl), benzyloxy group (BzlO), benzyl group (Bzl), benzoyl group (Bz), 3-nitro-2-pyridinesulfenyl group (Npys), 1-(4,4-dimethyl-2,6-diaxocyclohexylidene)ethyl group (Dde), 2,6-dichlorobenzyl group (2,6-DiCl-Bzl), 2-chlorobenzyloxycarbonyl group (2-Cl-Z), 2-bromobenzyloxycarbonyl group (2-Br-Z), benzyloxymethyl group (Bom), cyclohexyloxy group (cHxO), t-butoxymethyl group (Bum), t-butoxy group (tBuO), t-butyl group (tBu), acetyl group (Ac), and trifluoroacetyl group (TFA). From the viewpoint of handleability, Fmoc and Boc are preferable. Deprotecting methods with respect to these protecting groups are known, respectively, and those skilled in the art are able to perform deprotection appropriately.

### [Modifying group]

In the molecular probe precursor for use according to the present invention, the α-amino group at the N-terminus may be modified with a modifying group having no electric charge, from the viewpoint of canceling a positive charge of the α-amino group at the N-terminus thereby suppressing accumulation in kidneys of a molecular probe obtained by labeling or deprotecting the molecular probe precursor for use according to the present invention. For such modifying groups having no electric charge, groups described above as protective groups can be used, for example. Other examples of the modifying group having no electric charge include o-bromobenzyloxycarbonyl group, t-butyldimethylsilyl group, 2-chlorobenzyl (Cl-z) group, cyclohexyl group, cyclopentyl group, isopropyl group, pivalyl group, tetrahydropyran-2-yl group, and trimethylsilyl group. Among these, preferably, the modifying group is acetyl group, benzyl group, benzyloxymethyl group, o-bromobenzyloxycarbonyl group, t-butyl group, t-butyldimethylsilyl group, 2-chlorobenzyl group, 2,6-dichlorobenzyl group, cyclohexyl group, cyclopentyl group, isopropyl group, pivalyl group, tetrahydropyran-2-yl group, tosyl group, trimethylsilyl group, or trityl group. From the viewpoint of modifying the α-amino group at the N-terminus and canceling the positive electric charge, a protecting group different from the protecting group used for the amino group of the side chain of the lysine is preferred, and an acetyl group is preferred further.

### [Labeling compound]

A molecular probe precursor for use according to the present invention is a molecular probe precursor for labeling the amino group of the side chain of the lysine at the C-terminus with a labeling compound comprising an aromatic ring having a radioactive nuclide, and preferably it is a molecular probe precursor for labeling the amino group of the side chain of the lysine positioned on the C-terminus side in a molecular probe precursor as described herein consisting of a polypeptide of any one of the foregoing formulae (1) to (4) with the labeling compound.

Exemplary radioactive nuclides include ¹¹C, ¹³N, ¹⁵O, ¹⁸F, ⁶⁴Cu, ⁶⁷Ga, ⁶⁸Ga, ⁷⁵Br, ⁷⁶Br, ⁷⁷Br, 82Rb, ^{99m}Tc, ¹¹¹In, ¹²³I, ¹²⁴I, ¹²⁵I, and ¹³¹I. When PET is performed, exemplary preferred radioactive nuclides include a positron emission nuclide such as ¹¹C, ¹³N, ¹⁵O, ¹⁸F, ⁶²Cu, ⁶⁴Cu, ⁶⁸Ga, 75Br, ⁷⁶Br, 82Rb, and ¹²⁴I. When SPECT is performed, exemplary preferred radioactive nuclides include a γ-ray emission nuclide such as ⁶⁷Ga, ^{99m}Tc, ⁷⁷Br, ¹¹¹In, ¹²³I, and ¹²⁵I. Among these, radioactive halogen nuclides such as ¹⁸F, ⁷⁵Br, ⁷⁶Br, ⁷⁷Br, ¹²³I and ¹²⁴I are preferred further, and particularly preferred examples include ¹⁸F, ¹²³I and ¹²⁴I.

In the present specification, a "compound including an aromatic ring having a radioactive nuclide" indicates a compound that has a radioactive nuclide and either an aromatic hydrocarbon group or an aromatic heterocyclic group, and a preferred example thereof is a compound having a group represented by the following chemical formula (I).

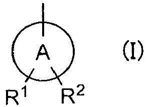

In the foregoing chemical formula (I), A represents an aromatic hydrocarbon group or an aromatic heterocyclic group. The aromatic hydrocarbon group preferably is an aromatic hydrocarbon group having 6 to 18 carbon atoms, and examples of the same include phenyl group, o-tolyl group, m-tolyl group, p-tolyl group, 2,4-xylyl group, p-cumenyl group, mesityl group, 1-naphthyl group, 2-naphthyl group, 1-anthryl group, 2-anthryl group, 9-anthryl group, 1-phenanthryl group, 9-phenanthryl group, 1-acenaphthyl group, 2-azulenyl group, 1-pyrenyl group, 2-triphenylenyl group, o-biphenylyl group, m-biphenylyl group, p-biphenylyl group, and terphenyl group. The aromatic heterocyclic group preferably is a 5 to 10-membered heterocyclic group having one or two of a nitrogen atom, an oxygen atom, or a sulfur atom, and examples of the same include triazolyl group, 3-oxadiazolyl group, 2-furyl group, 3-furyl group, 2-thienyl group, 3-thienyl group, 1-pyrrolyl group, 2-pyrrolyl group, 3-pyrrolyl group, 2-pyridyl group, 3-pyridyl group, 4-pyridyl group, 2-pyradyl group, 2-oxazolyl group, 3-isoxyazolyl group, 2-thiazolyl group, 3-isothiazolyl group, 2-imidazolyl group, 3-pyrazolyl group, 2-quinolyl group, 3-quinolyl group, 4-quinolyl group, 5-quinolyl group, 6-quinolyl group, 7-quinolyl group, 8-quinolyl group, 1-isoquinolyl group, 2-quinoxalynyl group, 2-benzofuryl group, 2-benzothienyl group, N-indolyl group, and N-carbazolyl group. A preferably is, among these, phenyl group, triazolyl group, or pyridyl group.

In the aforementioned chemical formula (I), R¹ represents a substituent that contains any one of ¹¹C, ¹³N, ¹⁵O, ⁶⁴Cu, ⁶⁷Ga, ⁶⁸Ga, ^{99m}Tc, and radioactive halogen, and the examples include radioactive halogen atoms, radiohalogenated C₁-C₃ alkyl group, radiohalogenated C₁-C₃ alkoxyl group and the like. Examples of the radioactive halogen include ¹⁸F, ⁷⁵Br, ⁷⁶Br, ⁷⁷Br, ¹²³I, ¹²⁴I, ¹²⁵I, and ¹³¹I. In the present specification, the "C₁-C₃ alkyl group" refers to an alkyl group that has 1 to 3 carbon atoms, and examples of the same include methyl group, ethyl group, and propyl group. In the present specification, the "radiohalogenated C₁-C₃ alkyl group" refers to an alkyl group that has 1 to 3 carbon atoms and in which a hydrogen atom is substituted with radioactive halogen. In the present specification, the "C₁-C₃ alkoxy group" refers to an alkoxy group that has 1 to 3 carbon atoms, and examples of the same include methoxy group, ethoxy group, and propoxy group. In the present specification, the "radiohalogenated C₁-C₃ alkoxy group" refers to an alkoxy group that has 1 to 3 carbon atoms and in which a hydrogen atom is substituted with radioactive halogen. From the viewpoint of quantitation, preferably R¹ is a substituent present on any of an ortho-position, a meta-position and a para-position, and more preferably R¹ is a substituent present on a meta-position or a para-position.

In the aforementioned chemical formula (I), R² represents a hydrogen atom or one or more substituents different from one or more substituents represented by R¹. R² may be a hydrogen atom or a substituent, but preferably, it is a hydrogen atom. In other words, in the aforementioned chemical formula (I), 'A preferably does not have a substituent other than R¹. In the case where R² represents a plurality of substituents, these substituents may be identical or different. Examples of the substituent include hydroxyl group, electron attractive groups, electron donative groups, C₁-C₆ alkyl groups, C₂-C₆ alkenyl groups, and C₂-C₆ alkynyl groups. Examples of the electron attractive group include cyano group, nitro group, halogen atoms, carbonyl group, sulfonyl group, acetyl group, and phenyl group. Examples of the halogen atom include fluorine atom, chlorine atom, bromine atom, and iodine atom. In the present specification, the "C₁-C₆ alkyl group" refers to an alkyl group having 1 to 6 carbon atoms, and examples of the same include methyl group, ethyl group, propyl group, isopropyl group, butyl group, isobutyl group, sec-butyl group, tert-butyl group, pentyl group, isopentyl group, and hexyl group. In the present specification, the "C₂-C₆ alkenyl group" refers to an alkenyl groups having 2 to 6 carbon atoms, and examples of the same include vinyl group, 1-propenyl group, 2-propenyl group, isopropenyl group, 1-butenyl group, 2-butenyl group, and 3-butenyl group. In the present specification, the "C₂-C₆ alkynyl group" refers to an alkynyl group having 2 to 6 carbon atoms, and examples of the same include ethynyl group, 1-propynyl group, 2-propynyl group, 1-butynyl group, 2-butynyl group, and 3-butynyl group. Among these, the hydroxyl group and the electron attractive group are preferred for the substituent.

Preferred examples of labeling compound including an aromatic ring having a radioactive nuclide include a compound having [¹⁸F]fluorobenzoyl group ([¹⁸F]FB), [¹²³I]iodobenzoyl group ([¹²³I]IB), [¹²⁴I]iodobenzoyl group ([¹²⁴I]IB), [¹²⁵I]iodobenzoyl group ([¹²⁵I]IB), [¹³¹I]iodobenzoyl group ([¹³¹I]IB), [¹²³I]indo p-hydroxyphenylpropionyl group, [¹²⁴I]dodo p-hydroxyphenylpropionyl group, [¹²⁵I]iodo p-hydroxyphenylpropionyl group or [¹³¹I]iodo p-hydroxyphenylpropionyl group, more preferably, [¹⁸F]N-succinimidyl 4-fluorobenzoate ([¹⁸F]SFB), [¹²³]N-succinimidyl 3-iodobenzoate ([¹²³I]SIB), [¹²⁴I]N-succinimidyl 3-iodobenzoate ([¹²⁴I]SIB), [¹²³I]iodo p-hydroxyphenylpropionic acid N-hydroxysuccinimide ester, and [¹²⁴I]iodo p-hydroxyphenylpropionic acid N-hydroxysuccinimide ester.

From the viewpoint of labeling with a metal radioactive isotope (metal nuclide) such as ⁶⁴Cu, ⁶⁷Ga, ⁶⁸Ga, ^{99m}Tc, ¹¹¹In and the like, in the molecular probe precursor for use according to the present invention, for example a chelate site that can be bonded to the metal radioactive isotope (metal nuclide) and/or a linker part serving for bonding to peptides can be bonded to the amino group of the side chain of the lysine at the C-terminus to be labeled. Examples of the chelate compound include diethylenetriaminepenta-acetic acid (DTPA), 6-hydrazinopyridine-3-carboxylic acid (HYNIC), tetraazacyclododecane tetraacetic acid (DOTA), dithisosemicarbazone (DTS), diaminedithiol (DADT), mercaptoacetylglycylglycylglycine (MAG3), monoamidemonoaminedithiol (MAMA), diamidedithiol (DADS), propylene diamine dioxime (PnAO) and the like.

In the molecular probe precursor for use in accordance with the present invention, from the viewpoint of affinity between a molecular probe obtained by labeling and deprotecting and pancreatic islets, preferably between the molecular probe and pancreatic β-cells, more preferably between the molecular probe and GLP-1 receptor of pancreatic islets, it is preferable that the diethylenetriaminepenta-acetic acid (DTPA) is not bonded to the amino group of the side chain of the lysine positioned on the C-terminus side, and more preferably, that a chelate site bondable to the metal radioactive isotope (metal nuclide) is not bonded. Examples of the other chelate compounds capable of forming the chelate site include 6-hydrazinopyridine-3-carboxylic acid (HYNIC), tetraazacyclododecane tetraacetic acid (DOTA), dithisosemicarbazone (DTS), diaminedithiol (DADT), mercaptoacetylglycylglycylglycine (MAG3), monoamidemonoaminedithiol (MAMA), diamidedithiol (DADS), propylene diamine dioxime (PnAO) and the like.

### [Method of Preparation of Molecular Probe as described herein]

The molecular probe as described herein can be prepared by labeling the molecular probe precursor as described herein, and thereafter, deprotecting the same by removing a protecting group. Exemplary radioactive nuclides used in labeling include ¹¹C, ¹³N, ¹⁵O, ¹⁸F, ⁶⁴Cu, ⁶⁷Ga, ⁶⁸Ga, ⁷⁵Br, ⁷⁶Br, ⁷⁷Br, ^{99m}Tc, ¹²³I, ¹²⁴I, ¹²⁵I, and ¹³¹I. Exemplary labeling procedures are as follows: when PET is performed as an imaging method, a positron emission nuclide such as ¹¹C, ¹⁵O, ¹⁸F or ¹²⁴I is labeled by a known method; and when SPECT is performed as an imaging method, a γ-ray emission nuclide such as ^{99m}Tc ¹²³I or ¹²⁵I is labeled by a known method. In the case of ¹⁸F, for example, it can be labeled by a method using [¹⁸F]SFB ([¹⁸F]N-succinimidyl 4-fluorobenzoate) or the like. In the case of ¹²³I or ¹²⁴I, it can be labeled by a method using [^{123/124}I]SIB ([^{123/124}I]N-succinimidyl 3-iodobenzoate), [^{123/124}I]iodo p-hydroxyphenylpropionic acid N-hydroxysuccinimide ester or the like. In the case of ¹²⁵I or ¹³¹I, it can be labeled by a method using [^{125/131}I]SIB ([^{125/131}I]N-succinimidyl 3-iodobenzoate), [^{125/131}I]iodo p-hydroxyphenylpropionic acid N-hydroxysuccinimide ester or the like. When the labeling is carried out by using a metal nuclide, the labeling is performed by using, for example, the above-described chelate compound. When the polypeptides of the foregoing formulae (1) to (4) are labeled by any of these methods, the following amino groups are labeled: the amino group of the side chain of the lysine at position 32 of the polypeptide of the foregoing formula (1), the amino group of the side chain of the lysine at position 31 of the polypeptide of the foregoing formula (2), the amino group of the side chain of the lysine at position 30 of the polypeptide of the foregoing formula (3), and the amino group of the side chain of the lysine at position 29 of the polypeptide of the foregoing formula (4). The labeling methods in the present invention are not limited to these methods. The deprotecting after the labeling can be carried out by a known method in accordance with the type of the protecting group. Therefore, another aspect of the present invention relates to a method for producing the molecular probe as described herein, the method including labeling and deprotecting the molecular probe precursor as described herein.

In the method of producing the molecular probe as described herein, the amino group of the side chain of the lysine at the C-terminus is labeled by a labeling compound including an aromatic ring having a radioactive nuclide. It is preferable that the labeling compound including the aromatic ring includes the group represented by the above chemical formula (I), wherein preferably R¹ represents a substituent comprising ¹⁸F, ⁷⁵Br, ⁷⁶Br, ⁷⁷Br, ¹²³I, ¹²⁴I, ¹²⁵I, or ¹³¹I.

In a method of producing a molecular probe as described herein, it is preferable that the labeling compound including an aromatic ring having a radioactive nuclide is a succinimidyl ester compound where the group represented by the above chemical formula (I) is bonded to succinimide via an ester bond, and more preferably, a succinimidyl ester compound represented by the following chemical formula (II).

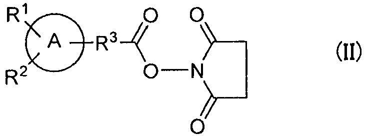

In the above chemical formula (II), A, R¹ and R² represent the groups or the substituents as those represented by the foregoing chemical formula (I). In chemical formula (II), R³ is preferably a bond, C₁-C₆ alkylene group or C₁-C₆ oxyalkylene group. In the present specification, "C₁-C₆ alkylene group" refers to an alkylene group having 1 to 6 carbon atoms, and the examples include a linear or branched alkylene group such as methylene group, ethylene group, propylene group, butylene group, pentyl group, hexyl group and the like. In the present specification, "C₁-C₆ oxyalkylene group" refers to an oxyalkynene group having 1 to 6 carbon atoms, and the examples include oxymethylene group, oxyethylene group, oxypropylene group, oxybutylene group, oxypentyl group and the like. From the viewpoint of affinity between a molecular probe and pancreatic islets, preferably between the molecular probe and pancreatic β-cells, more preferably between the molecular probe and GLP-1 receptor of pancreatic islets, R³ is preferably a bond, a methylene group or an ethylene group, and more preferably, a bond.

For the labeling compound including an aromatic ring having a radioactive nuclide, a compound having [¹⁸F]fluorobenzoyl group ([¹⁸F]FB), [¹²³I]iodobenzoyl group ([¹²³I]IB), [¹²⁴I]iodobenzoyl group ([¹²⁴I]IB), [¹²⁵I]iodobenzoyl group ([¹²⁵I]IB), [¹³¹I]iodobenzoyl group ([¹³¹I]IB), [¹²³I]iodo p-hydroxyphenylpropionyl group, [¹²⁴I]iodo p-hydroxyphenylpropionyl group, [¹²⁵I]iodo p-hydroxyphenylpropionyl group or [¹³¹I]iodo p-hydroxyphenylpropionyl group is preferred, and more preferably, [¹⁸F]N-succinimidyl 4-fluorobenzoate, [¹²³]N-succinimidyl 3-iodobenzoate, [¹²⁴I]N-succinimidyl 3-iodobenzoate, [¹²³I]iodo p-hydroxyphenylpropionic acid N-hydroxysuccinimide ester, and [¹²⁴I]iodo p-hydroxyphenylpropionic acid N-hydroxysuccinimide ester.

In a method of producing a molecular probe as described herein, the synthesis of the aforementioned labeling compound having a group represented by the chemical formula (I) and/or the aforementioned labeling compound including an aromatic ring having the radioactive nuclide may be carried out by using an automatic synthesizing device; and, the synthesis of a labeling compound having a group represented by the chemical formula (I) and/or a labeling compound including an aromatic ring having the aforementioned radioactive nuclide, and the labeling and deprotecting of the molecular probe precursor as described herein using the labeling compound may be carried out by one automatic synthesizing device.

### [Molecular Probe of the Present Invention]

Still another aspect of the present invention relates to a molecular probe for imaging of pancreatic islets, comprising or consisting of any one of the following polypeptides:
a polypeptide represented by any one of the following formulae (5) to (8),
   Z-DLSKQMEEEAVRLFIEWLKNGGPSSGAPPPSX-NH₂ (5) (SEQ ID NO. 5)
   Z-LSKQMEEEAVRLFIEWLKNGGPSSGAPPPSX-NH₂ (6) (SEQ ID NO. 6)
   Z-SKQMEEEAVRLFIEWLKNGGPSSGAPPPSX-NH₂ (7) (SEQ ID NO. 7)
   Z-KQMEEEAVRLFIEWLKNGGPSSGAPPPSX-NH₂ (8) (SEQ ID NO. 8)
   in the formulae (5) to (8), X represents a lysine residue having the amino group of the side chain labeled with a radioactive nuclide, where said amino group of the side chain of the lysine is bonded to a group comprising an aromatic ring represented by chemical formula (III) below, 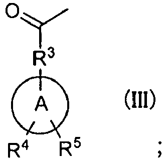
Z- indicates that the α-amino group at the N-terminus is unmodified or modified with a modifying group having no electric charge; and, -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group. The invention also extends to use of the molecular probe for imaging of pancreatic islets. The molecular probe may be obtained by the method for producing the molecular probe as described herein. With the molecular probe for imaging of the present invention, the noninvasive three-dimensional imaging of pancreatic islets, preferably the noninvasive three-dimensional imaging of pancreatic islets can be performed.

The molecular probe as described herein may have a configuration in which the following nuclide is bonded thereto: metal nuclides such as ⁶²Cu, ⁶⁴Cu, ⁶⁷Ga, ⁶⁷Ga, ⁶⁸Ga, ⁸²Rb, and ^{99m}Tc; and nuclides such as ¹¹C, ¹³N, ¹⁵O, ¹⁸F, ⁷⁵Br, ⁷⁶Br, ⁷⁷Br, ¹²³I, ¹²⁴I, ¹²⁵I, and ¹³¹I. It is preferable that radioactive nuclides such as ¹¹C, ¹³N, ¹⁵O, ¹⁸F, ⁷⁵Br, ⁷⁶Br, ⁷⁷Br, ¹²³I and ¹²⁴I are bonded, more preferably, radioactive nuclides such as ¹⁸F, ¹²³I and ¹²⁴I are bonded. When PET is performed, nuclides that emit positrons, such as ¹¹C, ¹³N, ¹⁵O, ¹⁸F, ⁶⁴Cu, ⁶⁸Ga, ^{75/76}Br, ⁸²Rb and ¹²⁴I, are preferred. When SPECT is performed, nuclides that emit single photons like γ-rays, such as ⁶⁷Ga, ^{99m}Tc, ⁷⁷Br and ¹²³I, are preferred. Molecular probes of the invention or for use in accordance with the invention have a nuclide selected from ¹⁸F, ⁷⁵Br, ⁷⁶Br, ⁷⁷Br, ¹²³I, ¹²⁴I, ¹²⁵I and ¹³¹I.

In the molecular probe as described herein, from the viewpoint of suppressing accumulation in kidneys, the α-amino group at the N-terminus may be modified with a modifying group having no electric charge. For the modifying group having no electric charge, the aforementioned examples can be employed. Among these, an acetyl group is preferred from the viewpoint of modifying the α-amino group at the N-terminus and canceling the positive electric charge.

In the molecular probe as described herein, it is preferable from the viewpoint of affinity between the molecular probe and pancreas, preferably between the molecular probe and pancreatic β-cells, more preferably between the molecular probe and GLP-1 receptor of pancreatic islets, that the DTPAis not bonded to the amino group of the side chain of the lysine on the C-terminus side, namely, any of the amino group of the side chain of the lysine at position 32 of the polypeptide of the foregoing formula (1), the amino group of the side chain of the lysine at position 31 of the polypeptide of the foregoing formula (2), the amino group of the side chain of the lysine at position 30 of the polypeptide of the foregoing formula (3), and the amino group of the side chain of the lysine at position 29 of the polypeptide of the foregoing formula (4). It is further preferable that a chelate site bondable to a metal radioactive isotope (metal nuclide) is not bonded to the amino group of the side chain of the foregoing lysine.

The α-amino group at the N-terminus of the molecular probe as described herein is unmodified, namely remains an amino group, or it is modified with a modifying group having no electric charge. From the viewpoint of increasing accumulation in pancreatic islets and suppressing accumulation in organs surrounding the pancreatic islets, it is preferable that the α-amino group at the N-terminus is unmodified, i.e., it remains an amino group. From the viewpoint of suppressing accumulation in kidneys, it is preferable that the α-amino group is modified with a modifying group having no electric charge. The modifying groups having no electric charge are aforementioned.

The molecular probe as described herein comprises a polypeptide that is used for imaging of pancreatic islets and that is represented by any of the foregoing formulae (5) to (8). The amino acid sequences of the polypeptides in the foregoing formulae (5) to (8) are the amino acid sequences described respectively in the SEQ ID NOS. 5-8 in the Sequence Listing. Further, the sequence of the amino acids at positions 1 to 31 in the foregoing formula (5) (SEQ ID NOS. 5) in the Sequence Listing coincide with the amino acid sequences of exendin(9-39) except for the α-amino group at the N-terminus bonded to a modifying group.

In the molecular probe as described herein, it is preferable that the amino group of the side chain of lysine labeled with the radioactive nuclide is bonded to a group including an aromatic ring represented by the following chemical formula (III).

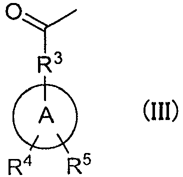

In the above chemical formula (III), A represents any of an aromatic hydrocarbon group or an aromatic heterocyclic group, the examples of the aromatic hydrocarbon group and the aromatic heterocyclic group are as described above. R⁵ represents a hydrogen atom or one or more substituents different from that represented by R⁴, and the examples are substantially same as those represented by R2.

In the above chemical formula (III), R⁴ represents a substituent containing ¹¹C, ¹³N, ¹⁵O, ¹⁸F, ⁷⁵Br, ⁷⁶Br, ⁷⁷Br, ¹²³I, ¹²⁴I, ¹²⁵I, or ¹³¹I. Examples of R⁴ include a ¹¹C atom, a ¹³N atom, a ¹⁵O atom, a ¹⁸F atom, a ⁷⁵Br atom, a ⁷⁶Br atom, a ⁷⁷Br atom, a ¹²³I atom, a ¹²⁴I atom, a ¹²⁵I atom, a ¹³¹I atom, a C₁-C₃ alkyl group substituted with [¹⁸F] fluorine, a C₁-C₃ alkoxy group substituted with [¹⁸F] fluorine, a C₁-C₃ alkyl group substituted with [¹²³I] iodine, a C₁-C₃ alkyl group substituted with [¹²⁴I] iodine, a C₁-C₃ alkyl group substituted with [¹²⁵I] iodine, a C₁-C₃ alkyl group substituted with [¹³¹I] iodine, a C₁-C₃ alkoxy group substituted with [¹²³I] iodine, a C₁-C₃ alkoxy group substituted with [¹²⁴I] iodine, a C₁-C₃ alkoxy group substituted with [¹²⁵I] iodine, and a C₁-C₃ alkoxy group substituted with [¹³¹I] iodine. In the molecular probe of the invention (or use thereof), R⁴ represents a substituent comprising ¹⁸F, ⁷⁵Br, ⁷⁶Br, ⁷⁷Br, ¹²³I, ¹²⁴I, ¹²⁵I, or ¹³¹I. Examples of the C₁-C₃ alkyl group include methyl group, ethyl group, propyl group and the like. Examples of the C₁-C₃ alkoxy group include methoxy group, ethoxy group, propoxy group and the like. From the viewpoint of quantitation, preferably R⁴ is a substituent present on any of an ortho-position, a meta-position and a para-position, and more preferably R⁴ is a substituent present on a meta-position or a para-position.

In the above chemical formula (III), similar to the case of the above chemical formula (II), preferably R³ is any of a bond, C₁-C₆ alkylene groups and C₁-C₆ oxyalkylene groups. In the molecular probe of the invention (or use thereof), R³ is any of a bond, a C₁-C₆ alkylene group and a C₁-C₆ oxyalkylene group. From the viewpoint of affinity with the molecular probe and pancreatic islets, preferably between the molecular probe and pancreatic β-cells, more preferably between the molecular probe and GLP-1 receptor of pancreatic islets, R³ is preferably a bond, a methylene group or an ethylene group, and more preferably, a bond.

It is preferable that a group including an aromatic ring represented by the chemical formula (III) has 7 to 20 carbon atoms, more preferably, 7 to 13 carbon atoms, and further preferably 7 to 9 carbon atoms, from the viewpoint of affinity with the molecular probe and pancreatic islets, preferably between the molecular probe and pancreatic β-cells, more preferably between the molecular probe and GLP-1 receptor of pancreatic islets.

In the molecular probe as described herein, it is preferable that a group having a radioactive nuclide to be bonded to the amino group of the side chain of the lysine labeled with a radioactive nuclide has carbon atoms of not more than 13, preferably, not more than 10, and further preferably not more than 9. And the lower limit is 1 for example, and preferably, 7. Therefore, a group having a radioactive nuclide to be bonded to the amino group of the side chain of a lysine labeled with a radioactive nuclide has carbon atoms of 1 to 13, more preferably 7 to 10, and further preferably 7 to 9.

### [Imaging Method]

Disclosed herein is a method of imaging pancreatic islets, wherein the method includes labeling a molecular probe precursor as described herein and subsequently deprotecting a protecting group. The method for imaging as described herein may include imaging pancreatic islets by using a molecular probe as described herein. From the viewpoint of application to examination and diagnosis, it is preferable that the method for imaging as described herein is a method of imaging pancreatic β-cells. Labeling and deprotecting of the precursor is as mentioned above, and the imaging of pancreatic islets is also as mentioned above. The method for imaging described herein may include determining a state of pancreatic islets based on the results of the imaging of pancreatic islets with use of the aforementioned molecular probe. Determining the state of pancreatic islets based on the results of the imaging of pancreatic islets with use of the molecular probe includes, for example, determining the presence/absence of pancreatic islets by analyzing an image of the imaging of pancreatic islets, and determining an increase/decrease in the amount of pancreatic islets.

Also disclosed is a method for imaging pancreatic islets, including detecting a signal of the molecular probe for imaging as described herein that has been administered to an analyte and/or a signal of the molecular probe for imaging as described herein that has been bound to pancreatic islets preliminarily. In the imaging method described herein, it is preferable that a signal of the molecular probe as described herein is detected from an analyte to which the molecular probe as described herein in an enough amount for imaging has been administered; it is further preferable that a signal of the molecular probe as described herein that has been bound to pancreatic islets preliminarily in an enough amount for imaging is detected.

Thus in a further embodiment the invention provides a method for imaging of pancreatic islets comprising:
(A) labeling and deprotecting the precursor of a molecular probe as described herein, wherein the labeling of the precursor comprises labeling the amino group of the side chain of the lysine at the C-terminus with a labeling compound comprising an aromatic ring having a radioactive nuclide, and imaging pancreatic islets by using the labeled and deprotected molecular probe for imaging of pancreatic islets, or
(B) detecting a signal of the molecular probe as described herein from an analyte to which the probe has been administered.

The detection of a signal of a molecular probe as described herein can be performed by, for example, the determination by means of PET and/or the determination by means of SPECT. The imaging method as described herein may include reconfiguring the detected signal so as to convert the same into an image, and further may include displaying the image. The measurement using PET and the measurement using SPECT include, for example, taking an image, and determining an amount of pancreatic islets.

The determination by means of SPECT includes, for example, determining, with use of a gamma camera, γ-rays emitted from an analyte (hereinafter, referred to also as "subject") to which the molecular probe as described herein has been administered. The determination with use of the gamma camera includes, for example, measuring radiation (γ-rays) emitted from the radioactive nuclide used for labeling the molecular probe as described herein during a certain time unit, and preferably includes determining a direction in which the radiation is emitted and a radiation dose during a certain time unit. The method for imaging as described herein further may include presenting the determined distribution of the molecular probe as described herein obtained by the measurement of the radiation as a cross-sectional image, and reconfiguring the obtained cross-sectional image. Examples of the subject include humans and mammals other than humans.

The determination by means of PET include, for example, simultaneously measuring a pair of annihilation radiations generated upon the coupling between a positron and an electron, with use of a detector for PET, from an analyte to which the molecular probe as described herein has been administered, and further may include figuring a three-dimensional distribution of positions of radioactive nuclide emitting positrons, based on the measurement results.

In the imaging method as described herein, the determination by means of X-ray CT or MRI may be performed, together with the determination by means of SPECT or the determination by means of PET. This makes it possible to obtain, for example, a fusion image obtained by fusion of an image obtained by SPECT or an image obtained by PET (functional image), with an image obtained by CT or an image obtained by MRI (morphological image).

The method for imaging as described herein may include administration of a molecular probe as described herein to a subject, and determination by any means such as PET or SPECT after a certain lapse time since the administration of the molecular probe. The method for imaging of pancreatic islets as described herein may include administering the molecular probe as described herein in an enough amount for obtaining a desired contrast for imaging. Further as described above, the method for imaging as described herein can be carried out after a certain lapse of time after administering the probe as described herein to an analyte, and thus, the method for imaging as described herein is not required to include administration of the molecular probe as described herein to a subject. The determination by PET or the like includes photographing of images, determination of pancreatic islets amount and the like. Examples of the subject include humans and mammals other than humans. The administration to a subject may be local administration or systemic administration. A path for administration may be determined appropriately according to a state of a subject and the like, and it may be, for example, intravenous, intraarterial, intradermal, and intraabdominal injection or infusion. The molecular probe as described herein preferably is administered together with a carrier. Examples usable as the carrier include aqueous solvents and non-aqueous solvents. Examples of the aqueous solvent include potassium phosphate buffer solution, physiologic saline, Ringer's solution, and distilled water. Examples of the non-aqueous solvent include polyethylene glycol, vegetable fats and oils, ethanol, glycerol, dimethyl sulfoxide, and propylene glycol. The amount of the molecular probe as described herein for imaging of pancreatic islets or determining an amount of pancreatic islets may be set to be, for example, not more than 1 µg. The time period from the administration to the determination may be decided appropriately according to, for example, a time that the molecular probe takes to bind to pancreatic islets, the type of the molecular probe, the decomposition time of the molecular probe, etc.

### [Method of Determining Amount of Pancreatic Islets]

Also disclosed herein is a method of determining an amount of pancreatic islets, and the method includes preparing a molecular probe as described herein by labeling and deprotecting a molecular probe precursor as described herein, and calculating an amount of pancreatic islets based on the result of imaging of pancreatic islets with use of the molecular probe. The method of determining an amount of pancreatic islets as described herein may include performing an imaging of pancreatic islets with use of the prepared molecular probe as described herein. Labeling and deprotecting are as mentioned above, and similarly, the imaging of pancreatic islets is as mentioned above. The calculation of an amount of pancreatic islets from results of imaging of pancreatic islets using the molecular probe may be performed by, for example, analyzing an image obtained by imaging of pancreatic islet. The quantitation of a subject of the imaging from results of the imaging can be performed easily by any person skilled in the art, using a calibration curve, an appropriately program, or the like. The method for determining an amount of pancreatic islets as described herein preferably is a method for determining an amount of pancreatic β-cells from the viewpoint of the application of the same to the examination and diagnosis.

Also disclosed is a method for determining an amount of pancreatic islets, including detecting a signal of the molecular probe for imaging as described herein from an analyte to which the molecular probe for imaging as described herein has been administered and/or a signal of the molecular probe for imaging as described herein that has been bound to pancreatic islets preliminarily, and calculating an amount of the pancreatic islets from the detected signal of the molecular probe for imaging.

Thus, in a further embodiment the invention provides a method for determining the amount of pancreatic islets, comprising:
(A) preparing a molecular probe for imaging of pancreatic islets by labeling and deprotecting the precursor of the molecular probe as described herein, wherein the labeling of the precursor comprises labeling the amino group of the side chain of the lysine at the C-terminus with a labeling compound comprising an aromatic ring having a radioactive nuclide; and
   calculating the amount of pancreatic islets based on the results of imaging of pancreatic islets with use of the molecular probe, or
(B) detecting a signal of the molecular probe as described herein for imaging of pancreatic islets from an analyte to which the probe has been administered; and calculating the amount of pancreatic islets based on the detected signal of the molecular probe for imaging of pancreatic islets.

The method for determining an amount of pancreatic islets as described herein may include presenting the calculated amount of pancreatic islets. Presenting the calculated amount of pancreatic islets includes, for example, storing the calculated amount of pancreatic islets or outputting the same to the outside. Outputting the same to the outside includes, for example, displaying the same on a monitor and printing the same.

### [Method of Prevention, Treatment, or Diagnosis of Diabetes]

Further disclosed herein is a method for prevention, treatment, or diagnosis of diabetes. Specifically, the method of prevention, treatment, or diagnosis of diabetes as described herein includes preparing a molecular probe for imaging of pancreatic islets by labeling and deprotecting a molecular probe precursor for imaging as described herein, performing an imaging of pancreatic islets by using the molecular probe for imaging of pancreatic islets, and diagnosing diabetes by determining a state of pancreatic islets on the basis of the obtained images of pancreatic islets and/or an amount of pancreatic islets, and the method may include prevention or treatment of diabetes on the basis of the diagnosis. As described above, in the diabetes developing process, the amount of pancreatic islets (particularly, the amount of pancreatic β-cells) decreases prior to the occurrence of glucose tolerance abnormalities, and therefore, when functional abnormalities are detected or there are subjective symptoms, diabetes has already reached the stage where it is too difficult to be treated. With the imaging method using the molecular probe precursor as described herein and/or the molecular probe as described herein, and/or the method for determining an amount of the pancreatic islets using the same, however, a decrease in the amount of the pancreatic islets and/or the amount of the pancreatic β-cells can be detected at an early stage, and further, new methods for prevention, treatment, and diagnosis of diabetes can be created. Examples of a subject on which prevention, treatment, and diagnosis of diabetes are carried out include humans and mammals other than humans. For example, the method for prevention of diabetes as described herein may include regularly determining the amount of pancreatic islets, and checking presence/absence of a tendency of a decrease in the amount of pancreatic islets. Further, the method for treatment of diabetes as described herein may include evaluating an effect of treatment such as medication and diet performed on a subject, focusing on a change in an amount of pancreatic islets. Further, the method of diagnosis of diabetes as described herein can include imaging of pancreatic islets or determining an amount of pancreatic islets, and comparing the results with a size or amount as a reference, or determining the stages of diabetes.

Preferably the method may be used for ultra-early diagnosis of diabetes. The method of ultra-early diagnosis of diabetes as described herein may include, for example, imaging pancreatic islets or determining an amount of pancreatic islets in comprehensive or ordinary medical examination by the method as described herein, and determining a state of the pancreatic islets on the basis of the obtained image of the pancreatic islets or the determined amount of the pancreatic islets. Further, a method for treatment of diabetes as described herein may include imaging pancreatic islets and/or determining an amount of pancreatic islets by the method as described herein, and evaluating functional recovery of the pancreatic islets on the basis of the obtained image of the pancreatic islets and/or the determined amount of the pancreatic islets.

Thus in a further embodiment, the invention provides the molecular probe as described herein for use in prevention, treatment, or *in vivo* diagnosis of diabetes, wherein in said prevention, treatment, or diagnosis said molecular probe is used in the imaging of pancreatic islets.

### [Kit of the Present Invention]

Still another aspect of the present invention also relates to a kit for preparing a molecular probe for imaging of pancreatic islets, and the kit includes a molecular probe precursor as described herein as well as a compound for labeling the precursor of a molecular probe for imaging of pancreatic islets, wherein the compound comprises an aromatic ring having a radioactive nuclide. Examples of embodiments of the kit of this aspect include a kit for preparing a molecular probe as described herein, a kit for performing the imaging method as described herein, a kit for performing the method for determining an amount of pancreatic islets as described herein, and a kit for prevention, treatment, or diagnosis of diabetes as described herein. Preferably, in each of these embodiments, the kit includes an instruction manual suitable for the embodiment.

The kit of the present invention includes a compound that is used for labeling the precursor of a molecular probe for imaging of pancreatic islets, which includes an aromatic ring having a radioactive nuclide. It is preferable that the compound including the aromatic ring is a compound having a group represented by the following chemical formula (IV).

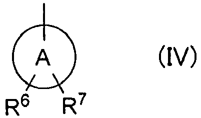

In the foregoing chemical formula (IV), A represents any of an aromatic hydrocarbon group or an aromatic heterocyclic group, the examples are as mentioned above. R⁷ represents a hydrogen atom or one or more substituents different from that represented by R⁶, and the examples are substantially same as those represented by R². R⁶ represents a substituent containing halogen or radioactive halogen. Examples of substituents containing halogen include a C atom, an N atom, an O atom, an F atom, a Br atom, an I atom, a C₁-C₃ alkyl group substituted with fluorine, a C₁-C₃ alkoxy group substituted with fluorine, a C₁-C₃ alkyl group substituted with iodine and a C₁-C₃ alkoxy group substituted with iodine. Examples of substituents containing radioactive halogen are substantially the same as those represented by R¹. From the viewpoint of quantitation, preferably R⁶ is a substituent present on any of an ortho-position, a meta-position and a para-position, and more preferably R⁶ is a substituent present on a meta-position or a para-position.

It is preferable that a labeling compound including an aromatic ring having a radioactive nuclide is a succinimidyl ester compound where the group represented by the above chemical formula (IV) is bonded to succinimide via an ester bonding, and more preferably, it is a succinimidyl ester compound represented by the following chemical formula (V).

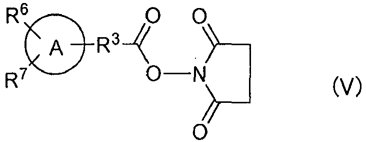

In the above chemical formula (V), examples ofA, R⁶ and R⁷ are substantially the same as those of the above chemical formula (IV). In the above chemical formula (V), like the chemical formulae (II) and (III), preferably R³ is any of a bond, a C₁-C₆ alkylene group and a C₁-C₆ oxyalkylene group. From the viewpoint of affinity with the molecular probe and pancreatic islets, preferably between the molecular probe and pancreatic β-cells, more preferably between the molecular probe and GLP-1 receptor of pancreatic islets, R³ is preferably a bond, a methylene group or an ethylene group, and more preferably, a bond.

For the labeling compound including an aromatic ring having a radioactive nuclide, a compound having any of [¹⁸F]fluorobenzoyl group, [¹²³I]iodobenzoyl group, [¹²⁴]iodobenzoyl group, [¹²⁵I]iodobenzoyl group, [¹³¹I]iodobenzoyl group, [¹²³I]iodo p-hydroxyphenylpropionyl group, [¹²⁴I]iodo p-hydroxyphenylpropionyl group, [¹²⁵I]iodo p-hydroxyphenylpropionyl group, and [¹³¹I]iodo p-hydroxyphenylpropionyl group is preferred. More preferably, [¹⁸F]N-succinimidyl 4-fluorobenzoate, [¹²³]N-succinimidyl 3-iodobenzoate, [¹²⁴I]N-succinimidyl 3-iodobenzoate, [¹²³I]iodo p-hydroxyphenylpropionic acid N-hydroxysuccinimide ester, and [¹²⁴I]iodo p-hydroxyphenylpropionic acid N-hydroxysuccinimide ester. The kit of the present invention may include an instruction manual reciting a method of labeling the molecular probe precursor as described herein that uses the aforementioned labeling compound and/or a compound for preparing the labeling compound.

The kit including the molecular probe precursor for imaging as described herein preferably further includes a starting material for the aforementioned labeling compound. Examples of the starting material for [¹⁸F]N-succinimidyl 4-fluorobenzoate include ethyl 4-(trimethylammonium triflate) benzoate, ethyl 4-(tosyloxy)benzoate, and ethyl 4-(methylsulfonyloxy)benzoate. Examples of the starting material for [^{123/124/125/131}I]N-succinimidyl 3-iodobenzoate include 2,5-dioxopyrrolidin-1-yl 3-(tributylstannyl)benzoate.

Still another aspect of the present invention also relates to a kit including the molecular probe as described herein. Examples of embodiments of the kit of the present invention include a kit for performing the imaging method as described herein, a kit for performing the method for determining an amount of pancreatic islets as described herein, and a kit for prevention, treatment, or diagnosis of diabetes as described herein. Preferably, in each of these embodiments, the kit includes an instruction manual suitable for the embodiment.

In the kit of the present invention, the molecular probe for imaging as described herein included in the kit preferably is in a form of a parenteral solution. Therefore, the kit of the present invention preferably includes a parenteral solution that contains the molecular probe for imaging as described herein. The parenteral solution may contain the molecular probe for imaging as described herein as an effective ingredient, and further, for example, a medicinal additive such as a carrier. In the present specification, the "medicinal additive" refers to a compound that has obtained authorization as a medicinal additive in the Japanese, U.S. and European pharmacopoeias. Examples of the carrier include aqueous solvents and non-aqueous solvents. Examples of the aqueous solvent include potassium phosphate buffer solution, physiologic saline, Ringer's solution, and distilled water. Examples of the non-aqueous solvent include polyethylene glycol, vegetable fats and oils, ethanol, glycerol, dimethyl sulfoxide, and propylene glycol. The kit of the present invention further may include a container for containing the molecular probe for imaging as described herein, and the container may be filled with the molecular probe for imaging as described herein or a parenteral solution that contains the molecular probe for imaging as described herein. Examples of the container include a syringe and a vial.

The kit of the present invention may further include, for example, a component used for preparing a molecular probe such as a buffer or an osmotic regulator, an instrument used in administration of a molecular probe, such as a syringe.

The kit including the molecular probe precursor as described herein further may include, for example, an automatic synthesizing device for synthesizing the labeling compound, and an instruction manual that describes a method for synthesizing a labeling compound having a group represented by the chemical formula (I) and/or a labeling compound including an aromatic ring having the aforementioned radioactive nuclide with use of the foregoing automatic synthesizing device for synthesizing the labeling compound. The automatic synthesizing device may be capable of synthesizing the labeling compound, and further, for example, capable of labeling and deprotecting the precursor of the molecular probe for imaging of pancreatic islets in which the synthesized labeling compound is used. The kit further may include, for example, a reagent containing a radioactive nuclide to be used in synthesizing the labeling compound. Examples of the reagent containing a radioactive nuclide include reagents containing radioactive isotopes such as ¹¹C, ¹³N, ¹⁵O, ¹⁸F, ⁶⁴Cu, ⁶⁷Ga, ⁶⁸Ga, ⁷⁵Br, ⁷⁶Br, ⁷⁷Br, ^{99m}Tc, ¹¹¹In, ¹²³I, ¹²⁴I, 1251 and ¹³¹I.

Still another aspect of the present invention relates to a kit that includes an automatic peptide synthesizing device for synthesizing the molecular probe precursor as described herein, and a labeling compound having a group represented by the chemical formula (I) and/or an automatic synthesizing device for synthesizing a labeling compound having a group represented by the chemical formula (I). The automatic synthesizing device may be capable of synthesizing the labeling compound, and further, for example, capable of labeling and deprotecting the molecular probe precursor in which the synthesized labeling compound is used. The kit may include an instruction manual that describes a method for synthesizing the molecular probe precursor as described herein. The instruction manual further may describe, for example, a method for synthesizing the labeling compound having a group represented by the chemical formula (I), a labeling method using the same, and a deprotecting method. The kit further may include a reagent containing radioactive nuclide to be used in synthesis of a labeling compound.

Also disclosed herein is a kit that includes the following: an automatic synthesizing device that performs the synthesis of the molecular probe precursor as described herein, the synthesis of the aforementioned labeling compound, and the labeling and deprotecting of the aforementioned molecular probe precursor for imaging of pancreatic islets in which the aforementioned labeling compound is used; and an instruction manual that describes a method for producing a molecular probe for imaging as described herein with use of the foregoing automatic synthesizing device. The instruction manual preferably describes, for example, a method for synthesizing a molecular probe precursor, a method for synthesizing the aforementioned labeling compound, and a method for labeling and deprotecting the molecular probe precursor in which the aforementioned labeling compound is used. The kit further may include a reagent containing radioactive nuclide to be used in synthesis of the labeling compound.

### [Reagent for Imaging]

Also disclosed herein is a reagent for imaging that contains the molecular probe as described herein. The reagent for imaging may contain the molecular probe as described herein as an effective ingredient, and further, a medicinal additive such as a carrier. The carrier is as described above.

Hereinafter, the present invention will be described further by way of Examples and Reference Examples. It should be noted that the present invention is, when interpreted, not limited to the following Examples.

In the description of the present application, the following abbreviations are used.
OBu: butyl ester group
Boc: butoxycarbonyl group
Trt: trityl group
Pdf: 2,2,4,6,7-pentamethyldihydrobenzofuran-5-sulfonyl group
Mmt: 4-methoxytrityl group
Fmoc: 9-fluorenylmethyloxycarbonyl group

### Examples

### (Example 1)

Using the molecular probe precursor of the above-described formula (1), which has a configuration in which protecting groups were bonded to an α-amino group at the N-terminus and the lysine residues at positions 4 and 19 of SEQ ID NO. 1 in the Sequence Listing, and in which the carboxyl group at the C-terminus is amidated, biodistribution of the same in a mouse was determined. First, a molecular probe for use according to the present invention was prepared in the following manner.

### [Preparation of Molecular Probe Precursor]

Polypeptide synthesis was performed by using an automatic peptide synthesizer (Model 433A) manufactured by Applied Biosystems, in accordance with the attached software. For the amino acids having functional groups at the side chains, Asp(OBu), Ser(OBu), Lys(Boc), Gln(Trt), Glu(OBu), Arg(Pbf), Asn(Trt) and Trp(Boc) were used respectively. For the lysines at positions 4 and 19, Lys(Mmt) were used. Rink Amide MBHA (0.125 mmol, 0.34 mmol/g) was employed as the starting resin, the amino acids were extended serially according to the sequence, thereby the polypeptide having the sequence of the following formula (9) was obtained. In the following formula (9), the protecting groups of the side chains other than Lys(Mmt) were not recited.
Fmoc-DLSK(Mmt)QMEEEAVRLFIEWLK(Mmt)NGGPSSGAPPPSK- resin (9) (SEQ ID NO. 9)
By a typical process using 1.5% TFA - 5% TIS - 93.55% CH₂Cl₂, the protecting groups (Mmt groups) of the side chains at the lysine residues at positions 4 and 19 were removed from the polypeptide of the above formula (9), and the amino groups of the side chains at the free lysine residues at positions 4 and 19 were Fmoc-bonded. Subsequently, removal of all of the protecting groups other than the Fmoc groups of the lysine residues at positions 4 and 19 and excision of peptide from the resin were carried out by a typical process using 92.5% TFA - 2.5% TIS - 2.5% H₂O - 2.5% ethanediol. After completion of the reaction, the carrier resin was removed by filtration, and dry ether was added thereto for precipitating the crude product, which was then filtered. The thus obtained crude product was purified in a linear gradient system of CH₃CN-H₂O containing 0.1% TFA, using a liquid chromatograph LC8A manufactured by Shimadzu Corp. (ODS column 3 cm × 25 cm). Then, intended fractions were collected by using a fraction collector, and thus the molecular probe precursor of the following formula (10) was obtained as a lyophilized white powder.
Fmoc-DLSK(Fmoc)QMEEEAVRLFIEWLK(Fmoc)NGGPSSGAPPPSK-NH₂(10) (SEQ ID NO. 10)

### [Preparation of Molecular Probe]

The thus obtained molecular probe precursor (640 µg) of the above-described formula (10) was dissolved in borate buffer (pH 7.8). [¹⁸F]SFB was added thereto so that pH of the reaction solution was adjusted to 8.5 to 9.0. Thus, the precursor was labeled. Thereafter, DMF and piperidine were added thereto so as to cause a deprotecting reaction, whereby the intended product (molecular probe in which the lysine residue at position 32 of SEQ ID NO. 5 was labeled) was obtained. In other words, the obtained molecular probe is a molecular probe of the following formula (11) (SEQ ID NO. 11) in which, in the amino acid sequence of SEQ ID NO. 5, [¹⁸F]FB (fluorobenzoyl group) is bound to the amino group of the side chain of the lysine at position 32, the carboxyl group at the C-terminus therein is amidated, and the α-amino group at the N-terminus is unmodified (amino group).

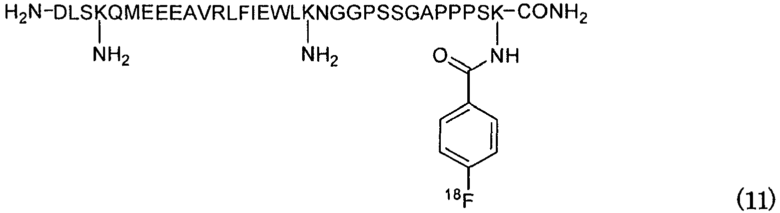

### [Biodistribution]

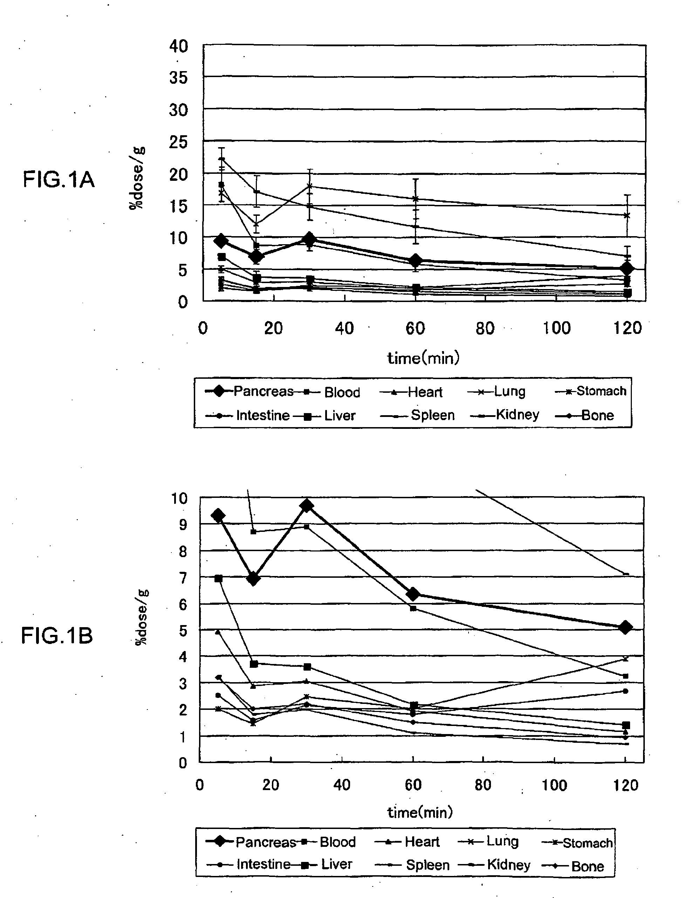

The molecular probe thus prepared (5.6 µCi) of the above formula (11) was administered to unanesthetized 6-week-old ddY mice (male, weight: 30g) by intravenous injection (through the tail vein). When 5 minutes, 15 minutes, 30 minutes, 60 minutes, and 120 minutes had passed since the administration, organs were dissected out of the mice, respectively (n=5). The weight and the radioactivity of each organ were determined, and an accumulation amount (%dose/g) of the molecular probe was calculated from the radioactivity per unit weight. Exemplary results are shown in the following Table 1, FIGS. 1A and 1B. FIG. 1A is a graph showing how the accumulation of the molecular probe in each organ varied with time, and FIG. 1B is a graph zooming in on FIG. 1A.

| (Table 1) | Time after administration | | | | |
|---|---|---|---|---|---|
| | 5min | 15min | 30min | 60min | 120min |
| Pancreas | 9.31 (0.57) | 6.93 (1.15) | 9.68 (0.92) | 6.33 (0.65) | 5.08 (1.23) |
| Blood | 18.12 (2.72) | 8.70 (1.18) | 8.87 (0.99) | 5.79 (1.15) | 3.23 (0.25) |
| Heart | 4.92 (0.49) | 2.86 (0.59) | 3.04 (0.35) | 1.94 (0.47) | 1.14 (0.1) |
| Lung | 16.79 (1.19) | 11.99 (1.39) | 17.96 (2.69) | 16.03 (3.16) | 13.32 (3.29) |
| Stomach | 2.00 (0.45) | 1.44 (0.3) | 2.46 (0.33) | 2.02 (0.33) | 3.88 (0.47) |
| Intestine | 2.52 (0.53) | 1.57 (0.27) | 2.13 (0.23) | 1.78 (0.15) | 2.69 (0.53) |
| Liver | 6.95 (0.39) | 3.72 (0.88) | 3.62 (0.25) | 2.17 (0.42) | 1.40 (0.23) |
| Spleen | 3.24 (0.45) | 1.79 (0.42) | 1.97 (0.32) | 1.09 (0.16) | 0.70 (0.09) |
| Kidney | 22.14 (1.75) | 17.11 (2.44) | 14.77 (2.12) | 11.64 (2.61) | 7.08 (1.47) |
| Bone | 3.18 (0.42) | 2.00 (0.35) | 2.20 (0.2) | 1.49 (0.27) | 0.93 (0.13) |

| | | | | | |
|---|---|---|---|---|---|
| Each numerical value indicates an average (SD) of 5 mice. | | | | | |

As shown in FIGS. 1A and 1B, the accumulation of the molecular probe prepared in Example 1 (the molecular probe of the above formula (11)) into the pancreas was 9.3 %dose/g at a point of 5 minutes after the administration, 6.9 %dose/g at a point of 15 minutes after the administration, and 9.7 %dose/g at a point of 30 minutes after the administration. During any of the time periods, the molecular probe prepared in Example 1 accumulated more in the pancreas than in the stomach, the intestines, the liver and the spleen as the organs adjacent to the pancreas. Particularly, during the time period from 5 minutes to 60 minutes after the administration, the accumulation amounts in the stomach and the intestines were as small as about 2 %dose/g, and during the time period from 5 minutes to 30 minutes after the administration, the accumulation amount in the pancreas during the same time period was approximately four times or more than the accumulation amounts in the stomach and the intestines. At or after 15 minutes since the administration, the accumulation amount in the liver was suppressed to 4 %dose/g or less. At or after 30 minutes since the administration, the accumulation amount in the pancreas was 2.5 times or more than the accumulation amount in the liver. This indicates that the molecular probe prepared in Example 1 accumulated specifically in the pancreas. Further, it was indicated that the radioactive accumulation in the bones was low and that it was not subjected to defluoridation metabolism in vivo. Therefore, the molecular probe of Example 1 (the molecular probe of the above formula (11)) is considered as suitable for the pancreatic β-cell imaging.

### (Reference Example 1)

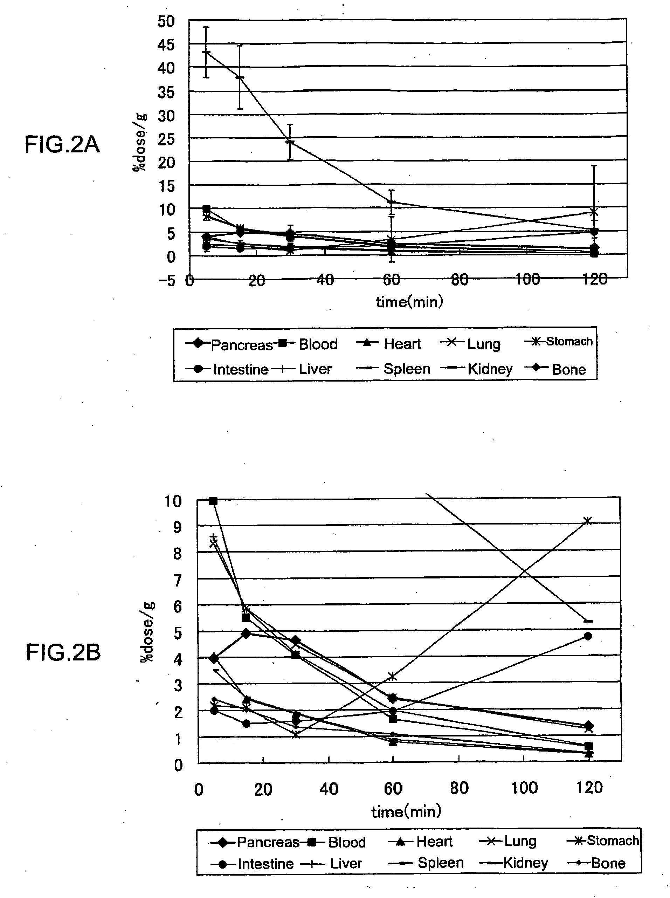

For Reference Example 1, a molecular probe was prepared from a molecular probe precursor of the following formula (12), which has a configuration in which protecting groups (Fmoc) were bonded to the α-amino group at the N-terminus and the lysine residue at position 19 of SEQ ID NO. 12 in the Sequence Listing and in which the carboxyl group at the C-terminus was amidated, and biodistribution of the same in a mouse was determined by using the molecular probe. In other words, biodistribution of a molecular probe (SEQ ID NO. 13) represented by the following formula (13) in a mouse was determined by using the same molecular probe in which [¹⁸F]FB (fluorobenzoyl group) was bonded to the amino group of the side chain of the lysine at position 4 and the carboxyl group at the C-terminus was amidated in the amino acid sequence of SEQ ID NO. 12. Preparation of the molecular probe precursor and the molecular probe and also determination of the biodistribution were carried out in the same manner as Example 1. Exemplary results are shown in the following Table 2, FIGS. 2A and 2B.
Fmoc-DLSKQMEEEAVRLFIEWLK(Fmoc)NGGPSSGAPPPS-NH₂ (12) SEQ ID NO. 12

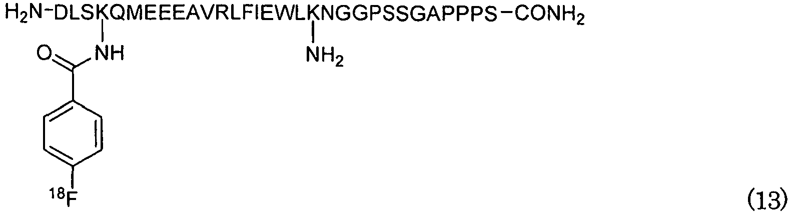

| (Table 2) | Time after administration | | | | |
|---|---|---|---|---|---|
| | 5min | 15min | 30min | 60min | 120min |
| Pancreas | 3.98 (0.27) | 4.92 (0.48) | 4.65 (1.83) | 2.42 (0.57) | 1.35 (0.37) |
| Blood | 9.95 (0.75) | 5.52 (0.26) | 4.08 (0.93) | 1.64 (0.14) | 0.57 (0.16) |
| Heart | 4.05 (0.34) | 2.43 (0.22) | 1.85 (0.67) | 0.79 (0.06) | 0.30 (0.08) |
| Lung | 8.33 (0.77) | 5.87 (0.47) | 4.49 (0.57) | 2.44 (0.49) | 1.24 (0.2) |
| Stomach | 2.18 (1.28) | 2.11 (1.08) | 1.09 (0.43) | 3.27 (4.79) | 9.08 (9.78) |
| Intestine | 1.99 (0.16) | 1.51 (0.1) | 1.58 (0.5) | 1.92 (1.19) | 4.73 (1.17) |
| Liver | 8.57 (0.80) | 5.82 (0.46) | 4.15 (0.62) | 1.96 (0.32) | 0.59 (0.22) |
| Spleen | 3.52 (0.36) | 2.48 (0.31) | 1.87 (0.47) | 0.86 (0.25) | 0.33 (0.08) |
| Kidney | 43.16 (5.40) | 37.86 (6.69) | 24.10 (3.82) | 11.25 (2.52) | 5.27 (1.88) |
| Bone | 2.41 (0.17) | 2.01 (0.18) | 1.36 (0.33) | 1.07 (0.73) | 0.32 (0.18) |

| | | | | | |
|---|---|---|---|---|---|
| Each numerical value indicates an average (SD) of 5 mice. | | | | | |

### (Reference Example 2)

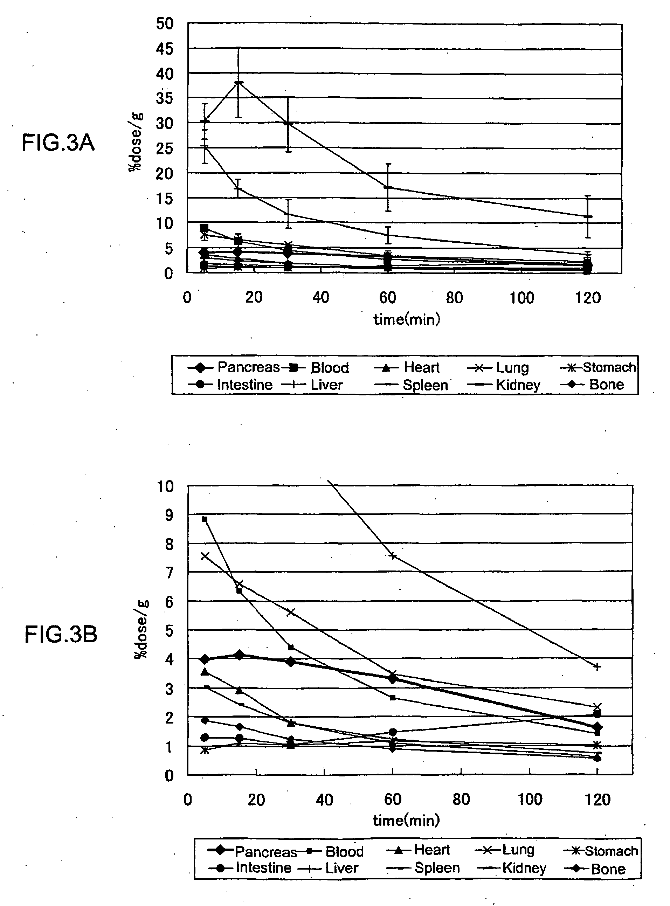

For Reference Example 2, a molecular probe was prepared from a molecular probe precursor of the following formula (14), which has a configuration in which protecting groups (Fmoc) were bonded to the N-terminus and the lysine residue at position 4 of SEQ ID NO. 14 in the Sequence Listing and in which the carboxyl group at the C-terminus was amidated, and biodistribution of the same in a mouse was determined by using the molecular probe. In other words, biodistribution of a molecular probe (SEQ ID NO. 15) represented by the following formula (15) in a mouse was determined by using the same molecular probe in which [¹⁸F]FB (fluorobenzoyl group) was bonded to the amino group of the side chain of the lysine at position 19 and the carboxyl group at the C-terminus was amidated in the amino acid sequence of SEQ ID NO. 14. Preparation of the molecular probe precursor and the molecular probe and also determination of the biodistribution were carried out in the same manner as Example 1. Exemplary results are shown in the following Table 3, FIGS. 3A and 3B.
Fmoc-DLSK(Fmoc)QMEEEAVRLFIEWLKNGGPSSGAPPPS-NH₂ (14) SEQ ID NO. 14

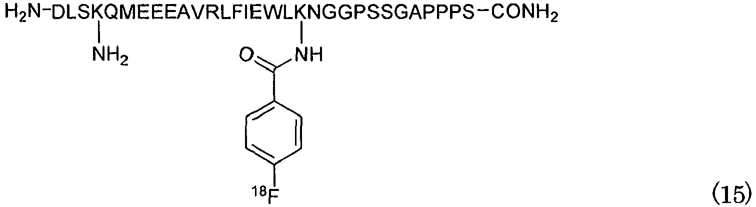

| (Table 3) | Time after administration | | | | |
|---|---|---|---|---|---|
| | 5min | 15min | 30min | 60min | 120min |
| Pancreas | 3.97 (0.89) | 4.13 (0.56) | 3.92 (0.51) | 3.32 (1.16) | 1.64 (0.15) |
| Blood | 8.84 (0.49) | 6.34 (1.41) | 4.40 (0.54) | 2.66 (0.74) | 1.42 (0.13) |
| Heart | 3.56 (0.38) | 2.92 (0.61) | 1.82 (0.21) | 1.09 (0.28) | 0.63 (0.08) |
| Lung | 7.56 (1.14) | 6.60 (0.47) | 5.62 (0.31) | 3.46 (0.56) | 2.33 (0.28) |
| Stomach | 0.87 (0.12) | 1.09 (0.2) | 1.04 (0.21) | 1.16 (0.54) | 1.00 (0.66) |
| Intestine | 1.29 (0.19) | 1.25 (0.36) | 1.04 (0.19) | 1.47 (0.25) | 2.09 (0.54) |
| Liver | 25.23 (3.40) | 16.81 (1.90) | 11.71 (2.74) | 7.56 (1.63) | 3.72 (0.58) |
| Spleen | 3.06 (0.79) | 2.42 (0.23) | 1.81 (0.34) | 1.22 (0.28) | 0.75 (0.23) |
| Kidney | 30.30 (3.53) | 38.04 (7.06) | 29.70 (5.57) | 17.14 (4.74) | 11.35 (4.10) |
| Bone | 1.87 (0.12) | 1.65 (0.21) | 1.23 (0.23) | 0.89 (0.16) | 0.56 (0.15) |

| | | | | | |
|---|---|---|---|---|---|
| Each numerical value indicates an average (SD) of 5 mice. | | | | | |

As shown in FIGS. 1A, 1B, 2A, 2B, 3A and 3B, the molecular probe prepared in Example 1 (the molecular probe of the above formula (11)) accumulated more in the pancreas and less in the stomach and intestines as the organs adjacent to the pancreas, in comparison with the molecular probe of Reference Example 1 prepared by using the molecular probe precursor of the above formula (12) or the molecular probe of Reference Example 2 prepared by using the molecular probe precursor of the above formula (14). Further, the molecular probe prepared in Example 1 accumulated less in the liver and the kidneys in comparison with the molecular probe of Reference Example 1 and the molecular probe of Reference Example 2. The accumulation amount of the molecular probe prepared in Example 1 in the kidneys was the half or less the accumulation amount in the kidneys of the molecular probe of any of Reference Examples 1 and 2. This indicates that the molecular probe prepared in Example 1 accumulated specifically in the pancreas.

Based on the accumulation amount obtained by the biodistribution experiments on the molecular probe in Example 1, the molecular probe in Reference Example 1 and the molecular probe in Reference Example 2, the ratio of pancreas/liver ('accumulation amount in pancreas' / 'accumulation amount in liver') for each probe is shown in Table 4 below, and the ratio of pancreas/kidney ('accumulation amount in pancreas' / 'accumulation amount in kidney') for each probe is shown in Table 5 below.

**(Table 4) Pancreas/Liver Ratio**

| | Time after administration | | | | |
|---|---|---|---|---|---|
| | 5min | 15min | 30min | 60min | 120min |
| Example 1 | 1.34 | 1.86 | 2.68 | 2.91 | 3.62 |
| Reference Example 1 | 0.46 | 0.85 | 1.12 | 1.24 | 2.28 |
| Reference Example 2 | 0.16 | 0.25 | 0.34 | 0.44 | 0.44 |
| Example 2 | 1.05 | 1.77 | 2.11 | 2.75 | 3.36 |

**(Table 5) Pancreas/Kidney Ratio**

| | Time after administration | | | | |
|---|---|---|---|---|---|
| | 5min | 15min | 30min | 60min | 120min |
| Example 1 | 0.42 | 0.40 | 0.66 | 0.54 | 0.72 |
| Reference Example 1 | 0.09 | 0.13 | 0.19 | 0.22 | 0.26 |
| Reference Example 2 | 0.13 | 0.11 | 0.13 | 0.19 | 0.14 |
| Example 2 | 0.41 | 0.48 | 0.54 | 0.51 | 0.75 |

As shown in the above Tables 4 and 5, the ratio of pancreas/liver and the ratio of pancreas/kidney for the molecular probe of Example 1 were high in comparison with the molecular probe of Reference Example 1 and the molecular probe of Reference Example 2. It was suggested that clear images of pancreas can be obtained at the time of imaging with the molecular probe of Example 1 where the ratio of accumulation amount in the pancreas to the surrounding organs of the pancreas is high and the accumulation amount in the surrounding organs of the pancreas is low.

By administering the molecular probe of the above Reference Example 1 to a mouse, a three-dimensional image of the pancreas of the mouse is obtained. Further, by administering the molecular probe of the above Reference Example 2 to a mouse, a noninvasive three-dimensional image of the pancreas of the mouse is obtained. As mentioned above, the molecular probe prepared in Example 1 by labeling the side chain of the lysine at the C-terminus accumulated more in the pancreas and accumulated less in the stomach and the intestines as the organs adjacent to the pancreas, in comparison with the molecular probe of Reference Example 1 prepared by using the molecular probe precursor of the formula (12) and the molecular probe of Reference Example 2 prepared by using the molecular probe precursor of the formula (14). This indicates that the molecular probe prepared in Example 1 enables a noninvasive three-dimensional imaging of pancreatic islets.

These results indicate that a molecular probe precursor for use according to the present invention enables a noninvasive three-dimensional imaging of pancreas in humans, particularly noninvasive three-dimensional imaging of pancreatic β-cells.

### [Blocking experiment]

Ablocking experiment was performed by using a molecular probe prepared in Example 1 (the molecular probe of the formula (11)). For the mice, 6-week-old ddY mice (male, weight: about 30g) were used.

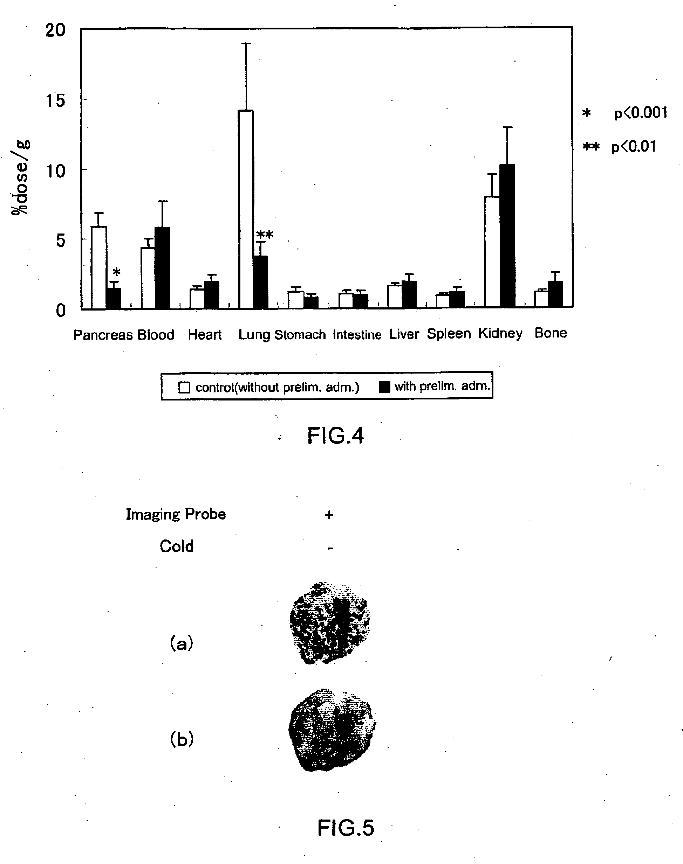

First, non-labeled exendin(9-39) (cold probe) was administered (0.1 mL of 0.5 mg/mL solution) preliminarily by intravenous injection to unanesthetized mice. At a point of 30 minutes after the foregoing preliminary administration, the prepared molecular probe of the formula (11) (5 µCi) was administered by intravenous injection. Then, at a time of 30 minutes after the administration of the molecular probe, the organs were dissected out, respectively (n=5). The weight and the radioactivity of each organ were determined, and an accumulation amount (%dose/g) was calculated from the radioactivity per unit weight. Exemplary results are shown in FIG. 4.

As a control, without preliminary administration of a cold probe, the prepared molecular probe (5 µCi) of the formula (11) was administered to unanesthetized mice by intravenous injection. Then, at a time of 30 minutes after the administration, the respective organs were dissected (n = 5). The weight and the radioactivity of each organ were determined, and an accumulation amount (%dose/g) of the probe was calculated from the radioactivity per unit weight. Exemplary results are shown in FIG. 4, together with the exemplary results for the case including the preliminary administration.

FIG. 4 is a graph showing an accumulation amount (%dose/g) for the case including the preliminary administration and an accumulation amount (%dose/g) for the control (without preliminary administration). As shown in FIG. 4, it was observed that, the binding with a receptor was inhibited by preliminary administration of a cold probe, whereby about 75% of the uptake of the molecular probe of the formula (11) was inhibited.

### [Two-Dimensional Imaging Analysis]

Two-dimensional imaging analysis was performed using transgenic mice that have a genetic background of ICR mice and express GFP (green fluorescent protein) under regulation of MIP (mouse insulin I gene promoter) (hereinafter these mice are referred to as "MIP-GFP mice"). For a molecular probe, the molecular probe of the formula (11) prepared in Example 1 was used.

The molecular probe thus prepared of the formula (11) was administered to unanesthetized MIP-GFP mice (male, weight: 20g) by intravenous injection (200 µL of about 171 µCi), and at a point of 30 minutes after the administration, the pancreases were dissected out of the mice (n=2). Sections were cut out of the dissected pancreases, and each section was placed on a slide glass, covered with a cover glass. Fluorescence and radioactivity (autoradiography) of each section were determined using an image analyzer (trade name: Typhoon 9410, produced by GE Health Care Inc.) (exposure time: 15 hours). Exemplary results of the same are shown in FIG. 5.

FIG. 5 shows exemplary results of the imaging analysis of the pancreas sections of the MIP-GFP mice at a point of 30 minutes after the administration of the molecular probe of the formula (11), where (a) indicates a fluorescence signal and (b) indicates a radioactivity signal. As shown in (a) and (b) of FIG. 5, a fluorescence GFP signal and a radioactivity signal were detected respectively by an image analyzer in each of the pancreas sections of the MIP-GFP mice. Further, as shown in (a) and (b) of FIG. 5, the localization of the radioactivity signal detected from the labeled molecular probe of the formula (11) was consistent with that of the GFP signal. From this, it was confirmed that the molecular probe of the formula (11) accumulated specifically in the pancreatic β-cells.

### [Three-Dimensional Imaging]

The prepared molecular probe of the formula (11) (89 µCi) was administered to anesthetized 6-week-old ddY mice (male, weight: 30g) by intravenous injection, and a three-dimensional imaging was carried out with the following PET device and under the following condition.
Imaging device: eXplore Vista (trade name, produced by GE Inc.)
Scan Mode: Static Scan
Reconstruction: 2DOSEM (Dynamic OS-EM)

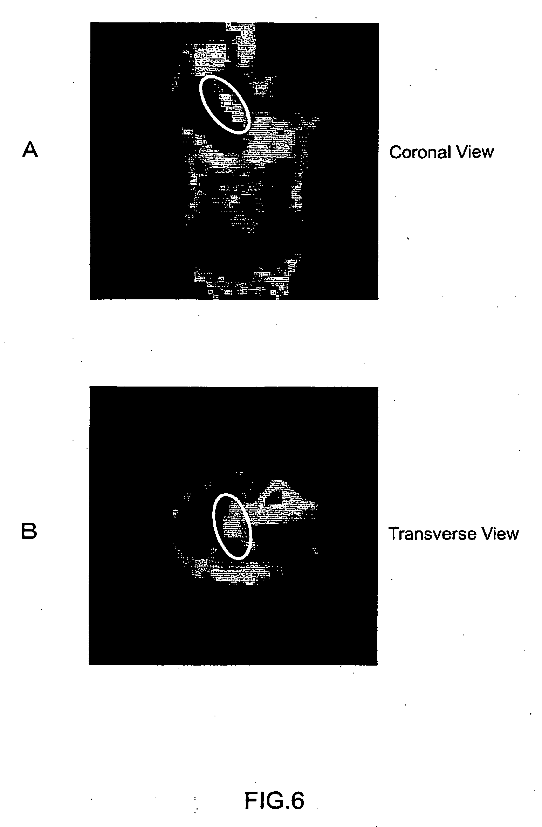

Exemplary results are shown in FIGS. 6A and 6B. The image was taken at a point of 30 minutes after the administration of the molecular probe (integrating time: 20 minutes). FIG. 6A shows a coronal view, and FIG. 6B shows a transverse view of the three-dimensional imaging. The white circles in FIGS. 6A and 6B indicate the positions of the pancreas. It should be noted that the images of FIGS. 6A and 6B are at the same contrast.

As shown in FIG. 6, the position of the pancreas was confirmed clearly and noninvasively with use of the molecular probe of the formula (11). In other words, it was confirmed that the molecular probe disclosed herein enables the noninvasive three-dimensional imaging of the pancreatic islet.

### (Example 2)

In the molecular probe precursor of the above-described formula (1), which has a configuration in which protecting groups were bonded to the lysine residues at positions 4 and 19 of SEQ ID NO. 1 in the Sequence Listing, and in which the carboxyl group at the C-terminus is amidated, using the molecular probe precursor of the above-described formula (16) in which the α-amino group at the N-terminus is acetylated, biodistribution of the same in a mouse was determined. First, the molecular probe was prepared in the following manner. For the protecting group of the lysine residue, Fmoc was used.
Ac-DLSK(Fmoc)QMEEEAVRLFIEWLK(Fmoc)NGGPSSGAPPPSK-NH₂ (16) (SEQ ID NO. 16)

### [Preparation of Molecular Probe]

The molecular probe precursor of the above formula (16) was prepared in the same manner as Example 1 except that the protecting group (Fmoc) of the α-amino group at the N-terminus was deprotected and acetylated. The obtained molecular probe precursor (540 µg) of the above formula (16) was dissolved in borate buffer (pH 7.8). [¹⁸F]SFB was added thereto so that pH of the reaction solution was adjusted to pH of 8.5 to 9.0. Thus, the precursor was labeled. Thereafter, DMF and piperidine were added thereto so as to cause a deprotecting reaction, whereby the intended product (molecular probe where the lysine residue at position 32 of SEQ ID NO. 5 is labeled) was obtained. In other words, the obtained molecular probe is a molecular probe of the following formula (17) (SEQ ID NO. 17) in which, in the amino acid sequence of SEQ ID NO. 5, [¹⁸F]FB (fluorobenzoyl group) is bound to the amino group of the side chain of the lysine at position 32 in the amino acid sequence, the α-amino group at the N-terminus is acetylated, and the carboxyl group at the C-terminus is amidated. In the following formula (17), Ac indicates that the α-amino group at the N-terminus is acetylated.

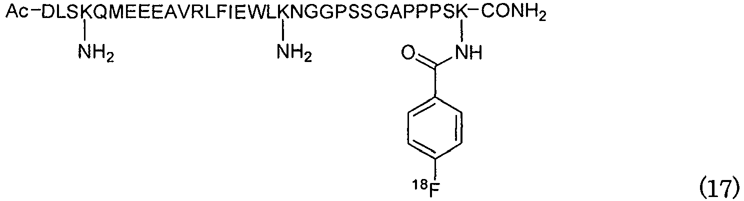

### [Biodistribution]

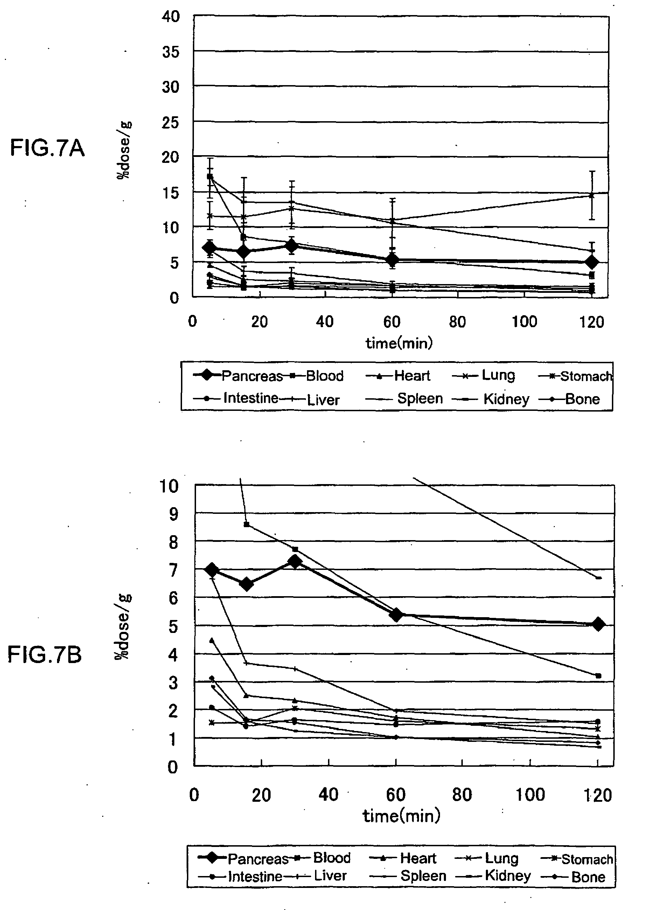

The probe of the above formula (17) thus prepared (7 µCi) was administered to unanesthetized 6-week-old ddY mice (male, weight: 30g) by intravenous injection (through the tail vein). When 5 minutes, 15 minutes, 30 minutes, 60 minutes, and 120 minutes had passed since the administration, organs were dissected out of the mice, respectively (n=5). The weight and the radioactivity of each organ were determined, and an accumulation amount (%dose/g) of the probe was calculated from the radioactivity per unit weight. Exemplary results are shown in the following Table 6, FIGS. 7A and 7B. FIG. 7A is a graph showing how the accumulation of the molecular probe in each organ varied with time, and FIG. 7B is a graph zooming in on FIG. 7A.

| (Table 6) | Time after administration | | | | |
|---|---|---|---|---|---|
| | 5min | 15min | 30min | 60min | 120min |
| Pancreas | 6.98 (1.10) | 6.47 (1.61) | 7.30 (1.29) | 5.37 (0.97) | 5.05 (0.55) |
| Blood | 17.02 (1.2) | 8.59 (2.06) | 7.74 (0.86) | 5.49 (1.42) | 3.20 (0.42) |
| Heart | 4.46 (0.28) | 2.51 (0.62) | 2.33 (0.31) | 1.71 (0.53) | 1.04 (0.2) |
| Lung | 11.53 (1.97) | 11.44 (2.83) | 12.65 (2.99) | 11.06 (2.53) | 14.52 (3.45) |
| Stomach | 1.52 (0.26) | 1.53 (0.4) | 2.06 (0.43) | 1.60 (0.18) | 1.34 (0.15) |
| Intestine | 2.06 (0.07) | 1.40 (0.35) | 1.63 (0.28) | 1.45 (0.2) | 1.58 (0.47) |
| Liver | 6.64 (1.09) | 3.65 (0.68) | 3.46 (0.71) | 1.96 (0.34) | 1.50 (0.19) |
| Spleen | 2.81 (0.44) | 1.59 (0.53) | 1.26 (0.18) | 1.01 (0.30) | 0.69 (0.13) |
| Kidney | 16.88 (2.86) | 13.50 (3.44) | 13.50 (2.96) | 10.59 (3.56) | 6.70 (1.21) |
| Bone | 3.11 (0.14) | 1.66 (0.36) | 1.54 (0.33) | 1.03 (0.34) | 0.84 (0.18) |

| | | | | | |
|---|---|---|---|---|---|
| Each numerical value indicates an average (SD) of 5 mice. | | | | | |

As shown in FIGS. 7A and 7B, the accumulation of the molecular probe of the above formula (17) prepared in Example 2 into the pancreas was 7.0 %dose/g at a point of 5 minutes after the administration, 6.5 %dose/g at a point of 15 minutes after the administration, and 7.3 %dose/g at a point of 30 minutes after the administration. During any of the time periods, the molecular probe of the above formula (17) prepared in Example 2 accumulated more in the pancreas than in the stomach and the intestines as the organs adjacent to the pancreas. Particularly, during any of the time periods, the accumulation amounts in the stomach were as small as about 2 %dose/g, the accumulation amounts in the intestines were as small as about 1.7 %dose/g, and the accumulation amount in the pancreas was three times or more than the accumulation amounts in the stomach and intestines during this time period. At or after 15 minutes since the administration, the accumulation in the liver was suppressed to not more than 4 %dose/g. At or after 30 minutes since the administration, the accumulation amount in the pancreas was twice or more than the accumulation amount in the liver. This indicates that the molecular probe of the above formula (17) prepared in Example 2 accumulated specifically in the pancreas. Further, it was suggested that the radioactive accumulation in the bones was small and that it was not subjected to defluoridation metabolism in vivo. Therefore, the molecular probe of the above formula (17) is considered as suitable for the pancreatic β-cell imaging.

Furthermore, as shown in FIGS. 1A, 1B and FIGS. 7A and 7B, accumulation in kidneys of the molecular probe of Example 2 (the molecular probe of the above formula (17)) in which the α-amino group at the N-terminus was acetylated was suppressed in comparison with the case of molecular probe of Example 1 (the molecular probe of the above formula (11)) in which the α-amino group at the N-terminus was not acetylated.

Based on the accumulation amount obtained by the biodistribution experiments on the molecular probe in Example 2, the ratio of pancreas/liver ('accumulation amount in pancreas' / 'accumulation amount in liver') is shown in Table 4 above, and the ratio of pancreas/kidney ('accumulation amount in pancreas' / 'accumulation amount in kidney') is shown in Table 5 above. As shown in the above Tables 4 and 5, the ratio of pancreas/liver and the ratio of pancreas/kidney of the molecular probe of Example 2 were higher in comparison with the molecular probe of Reference Example 1 and the molecular probe of Reference Example 2. It was suggested that clear images of pancreas can be obtained at the time of imaging with the molecular probe of Example 2 where the ratio of accumulation amount in the pancreas to the surrounding organs is high and the accumulation amounts in the surrounding organs of the pancreas are low.

As shown in FIGS. 2A, 2B, FIGS. 3A, 3B and FIGS. 4A and 4B, the molecular probe of the above formula (17) prepared according to Example 2 accumulated in the pancreas more while the accumulation amounts in the stomach and the intestines as organs adjacent to the pancreas were small in comparison with the cases of molecular probe of the above Reference Example 1 and the molecular probe of the above Reference Example 2. Further, the molecular probe of the above formula (17) prepared according to Example 2 accumulated in kidneys less in comparison with the case of molecular probes of the Reference Examples 1 and 2. The accumulation amount in kidneys of the molecular probe prepared according to Example 2 was the half or less than the accumulation amount in kidneys of the molecular probe of any of Reference Example 1 (the molecular probe of the above formula (13)) and Reference Example 2 (the molecular probe of the above formula (15)). This suggests that the molecular probe of the above formula (17) prepared according to Example 2 accumulated particularly in the pancreas. Therefore, it was suggested that the molecular probe of the above formula (17) prepared according to Example 2 enabled a noninvasive three-dimensional imaging of pancreatic islets.

The foregoing results suggested that the molecular probe precursor for use according to the present invention enables noninvasive three-dimensional imaging of the pancreas, and particularly, noninvasive three-dimensional imaging of pancreatic β-cells, in humans.

### (Example 3)

### [Binding Assay]

Labeling and deprotecting of the molecular probe precursor of the above formula (10) prepared in Example 1 were carried out in the same manner as Example 1 except that the [¹⁸F]SFB was replaced by [¹²⁷I]N-succinimidyl 3-iodobenzoate ([¹²⁷I]SIB), thereby obtaining a molecular probe (SEQ ID NO. 18) of the formula (18) below.

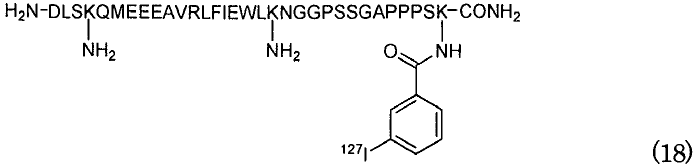

Pancreatic islets isolated from a mouse was recovered in a 50 ml-tube and after centrifugation (2000 rpm, 2 minutes), it was washed once with 20 mL of cold PBS. To which, 15 mL of trypsin-EDTA (which was prepared by adding 12 mL of PBS-containing 0.53mM EDTA (pH: 7.4 (NaOH)) to 3 mL of trypsin-EDTA (0.05% / 0.53mM) was added, and incubating with shaking at 37°C for one minute, it was placed immediately on ice. Subsequently, after pipetting vigorously 20 times with a 10 mL pipette dropper without foaming, the cold PBS was added so that the final amount would be 30 mL. After centrifugation (3000 rpm, 2 minutes), it was washed twice with 30 mL of cold PBS. The supernatant was removed to obtain pancreatic islet cells sample. The obtained pancreatic islet cells sample was reserved at -80°C.

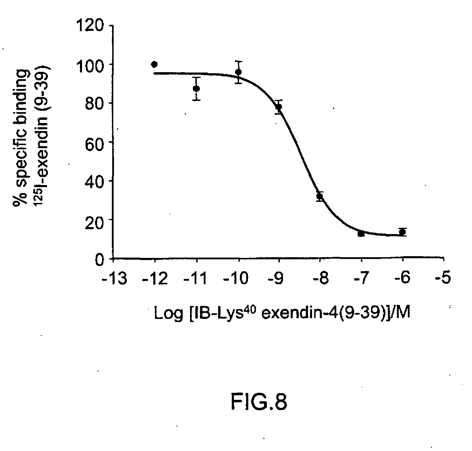

The pancreatic islet cells sample was suspended in a buffer (20mM, HEPES (pH: 7.4), 1 mM MgCl₂, 1mg/ml bacitracin, 1 mg/ml BSA) so as to make 100 µL/tube. Then, 880 µL of the buffer and 10 µL of a solution including the molecular probe of the above formula (18) (final concentration of molecular probe: 0,1×10⁻⁶ to 1x10⁻¹² M), and 10 µL of a solution including [¹²⁵I] labeled Exendin (9-39) (prepared by adding 90 µL of a buffer to 10 µL of [¹²⁵I]Bolton-Hunter labeled Exendin (9-39) (product code: NEX335, 1.85 MBq/mL = 50 µCi/mL, 22.73 pmol/mL = 76.57 ng/mL, manufactured by Perkin Elmer) were added thereto, which was incubated for 60 minutes at room temperature. Here, the final concentration of the [¹²⁵I] labeled Exendin (9-39) was set to 0.05 µCi/tube. Next, after B/F separation by aspirating with use of an aspirator to which a moistened glass fiber filter (Whatman GF/C filter) was attached, the filter was washed three times with 5 ml of an ice-cooled PBS. The filter was set in the tube, and the radioactivity measurement was carried out with a gamma counter. The results are shown in FIG. 8.

FIG. 8 is a graph showing exemplary results of analysis with a SigmaPlot11 (trade name). As shown in FIG. 8, the molecular probe of the above formula (18) inhibited in a concentration-dependent manner the binding between the GLP-1R and the [¹²⁵I] labeled Exendin (9-39). The IC₅₀ of the molecular probe of the above formula (18) was 3.52 × 10⁻⁹ M, and thus the molecular probe of the above formula (18) exhibited a high affinity with respect to the GLP-1 receptor of pancreatic islets. Moreover, the IC₅₀ of the molecular probe of the above formula (18) was approximate to that of Exendin (9-39) (IC₅₀: 1.4 × 10⁻⁹M), and thus, with respect to the GLP-1 receptor of pancreatic islets, the molecular probe of the above formula (18) is considered to have an affinity comparable to that of Exendin (9-39) as an antagonist for GLP-1 receptor of pancreatic islets.

Next, using a molecular probe (SEQ ID NO. 19) of the following formula (19), which has a configuration in which the amino group of the side chain of the lysine residue at position 32 is labeled with 3-[¹²⁵I]iodobenzoyl group (hereinafter, referred to also as "[¹²⁵I]IB label"), the carboxyl group at the C-terminus is amidated and the α-amino group at the N-terminus is not acetylated in the amino acid sequence of SEQ ID NO. 5 in the Sequence Listing, determination of biodistribution of the same in a mouse and a two-dimensional analysis were carried out. The molecular probe of the following formula (19) was prepared in the same manner as in Example 1 except that [¹²⁵I]N-succinimidyl 3-iodobenzoate (SIB) was used in place of [¹⁸F]SFB.

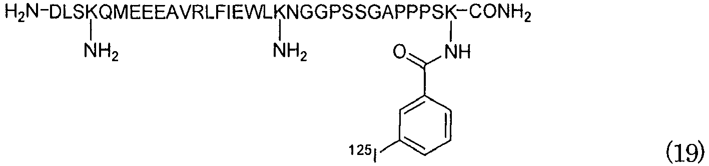

### [Biodistribution]

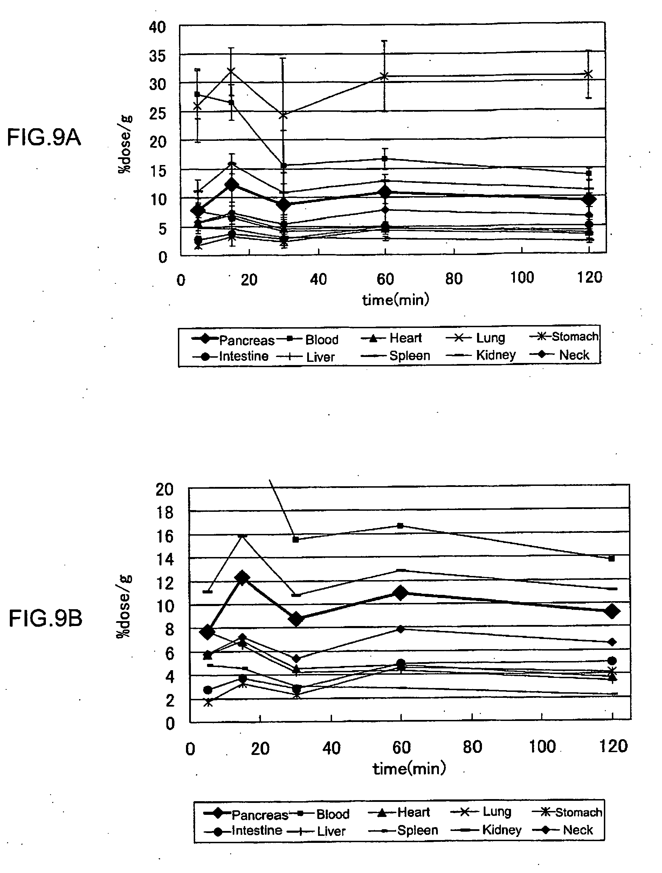

The molecular probe thus prepared (1 µCi) of the formula (19) was administered to unanesthetized 6-week-old ddY mice (male, weight: about 30g) by intravenous injection (through the tail vein). When 5 minutes, 15 minutes, 30 minutes, 60 minutes, and 120 minutes had passed since the administration, organs were dissected out of the mice, respectively (n=5). The weight and the radioactivity of each organ were determined, and an accumulation amount (%dose/g) was calculated from the radioactivity per unit weight. Exemplary results are shown in the following Table 7, FIGS. 9A and 9B. FIG. 9A is a graph showing how the accumulation of the molecular probe in each organ varied with time, and FIG. 9B is a graph zooming in on FIG. 9A.

| (Table 7) | Time after administration | | | | |
|---|---|---|---|---|---|
| | 5min | 15min | 30min | 60min | 120min |
| Pancreas | 7.73 (2.18) | 12.28 (3.74) | 8.73 (3.65) | 10.90 (1.96) | 9.23 (1.19) |
| Blood | 27.91 (4.16) | 26.44 (3.06) | 15.53 (6.05) | 16.65 (1.77) | 13.67 (0.99) |
| Heart | 5.74 (1.38) | 6.92 (0.79) | 4.56 (2.00) | 4.80 (0.17) | 3.66 (0.49) |
| Lung | 25.98 (6.37) | 31.88 (4.17) | 24.29 (9.94) | 30.96 (6.2) | 31.02 (4.18) |
| Stomach | 1.72 (0.52) | 3.26 (1.65) | 2.30 (1.04) | 4.58 (0.96) | 4.15 (1.70) |
| Intestine | 2.76 (0.56) | 3.74 (0.41) | 2.85 (1.18) | 4.94 (0.84) | 5.00 (1.23) |
| Liver | 7.71 (1.23) | 6.54 (0.66) | 4.18 (1.72) | 4.32 (0.41) | 3.42 (0.27) |
| Spleen | 4.86 (0.50) | 4.61 (0.70) | 3.03 (1.05) | 2.80 (0.39) | 2.20 (0.36) |
| Kidney | 11.09 (1.94) | 15.83 (1.69) | 10.80 (4.50) | 12.86 (1.13) | 11.14 (1.40) |
| Neck | 5.81 (1.93) | 7.26 (1.83) | 5.41 (1.67) | 7.80 (2.12) | 6.58 (2.35) |

| | | | | | |
|---|---|---|---|---|---|
| Each numerical value indicates an average (SD) of 5 mice. | | | | | |

As shown in FIGS. 9A and 9B, the accumulation of the molecular probe of the formula (19) prepared in Example 3 into the pancreas was 7.7 %dose/g at a point of 5 minutes after the administration, 12.3 %dose/g at a point of 15 minutes after the administration, 8.7 %dose/g at a point of 30 minutes after the administration, and 10.9 %dose/g at a point of 60 minutes after the administration. During any of the time periods, the molecular probe of the formula (19) accumulated more in the pancreas than in the stomach and the intestines as the organs adjacent to the pancreas. Particularly, during the time period from 5 minutes to 30 minutes after the administration, the accumulation amounts in the stomach and intestines were as small as about 4 %dose/g, and the accumulation amount in the pancreas was three times or more than the accumulation amounts in the stomach and intestines during this time period. At or after 30 minutes since the administration, the accumulation amount of the molecular probe of the formula (19) in the liver was not more than 5 %dose/g. This indicates that the molecular probe of the formula (19) accumulated specifically in the pancreas. Further, no great change was seen in the accumulation in the neck, and this suggests that the molecular probe of the formula (19) was not subjected to deiodization metabolism in vivo. Therefore, the molecular probe of the formula (19) is considered suitable for the pancreatic β-cell imaging, particularly noninvasive pancreatic β-cell imaging.

### [Two-Dimensional Imaging Analysis]

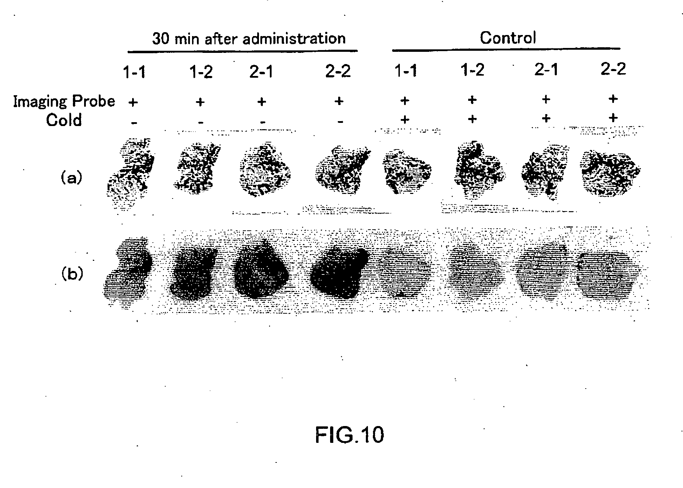

The molecular probe of the formula (19) (5 µCi) was administered to unanesthetized MIP-GFP mice (male, weight: 20g) by intravenous injection, and at points of 30 minutes and 60 minutes after the administration, the pancreases were dissected out of the mice, respectively (n=2). Sections were cut out of the dissected pancreases, and each section was placed on a slide glass, covered with a cover glass. Fluorescence and radioactivity (autoradiography) of each section were determined using an image analyzer (trade name: Typhoon 9410, produced by GE Health Care Inc.) (exposure time: 18 hours). Exemplary results of the same are shown in FIG. 10.

As a control, a commercially-available Exendin (9-39) where the labeling group is not bound (cold probe) was administered preliminarily to unanesthetized MIP-GFP mice (male, weight: 20g) by intravenous injection (50 µg / 100 µL). At a point of 30 minutes after the foregoing preliminary administration, the prepared molecular probe of the formula (19) (5 µCi) was administered by intravenous injection. Then, at a time of 30 minutes after the administration of the molecular probe of the above formula (19), the pancreases were dissected out (n=2). Sections were cut out of the dissected pancreases, and fluorescence and radioactivity of each section were determined in the same manner as described above. One of exemplary results of the same is shown in FIG. 10 together with an exemplary result without the preliminary administration.

FIG. 10 shows exemplary results of the imaging analysis of the pancreas sections of the MIP-GFP mice to which the molecular probe of the formula (19) was administered. The images shown therein are images showing a fluorescence signal (a) and a radioactivity signal (b) of each of the sections at the point of 30 minutes after the administration of the molecular probe of the formula (19).

As shown in (a) and (b) of FIG. 10, a fluorescence GFP signal and a radioactivity signal were detected by an image analyzer in each of the pancreas sections of the MIP-GFP mice. Further, the localization of the radioactivity signal detected from the labeled molecular probe of the formula (19) was consistent with that of the GFP signal. From this, it was confirmed that the molecular probe of the formula (19) accumulated specifically in the pancreatic β-cells.

As shown in FIG. 10(b), substantially no radioactivity signal was detected from the control section to which the cold probe had been administered preliminarily. From this, it was observed that, the binding with a GLP-1 receptor was inhibited by preliminary administration of a cold probe, whereby the uptake of the molecular probe of the formula (19) was inhibited. From this, it was confirmed that the molecular probe of the formula (19) was bound to the GLP-1 receptor of a pancreatic β-cell.

Here, all of ¹²⁵I, ¹²³I, and ¹³¹I were γ-ray emitting nuclides. Still further, ¹²⁵I and ¹²³I have the same numbers of nuclear spins. In view of these, it can be presumed that even a molecular probe obtained by replacing the radioactive iodine atom (¹²⁵I) used in the labeling of the molecular probe of the formula (19) with ¹²³I or ¹³¹I will exhibit behaviors substantially identical to those of the molecular probe of the formula (19). Further, it also can be presumed that even a molecular probe obtained by the radioactive iodine atom (¹²⁵I) with ¹²⁴I will exhibit behaviors substantially identical to those of the molecular probe of the formula (19). Thus, it was suggested that using the molecular probe obtained by replacing ¹²⁵I of the molecular probe of the formula (19) with ¹²³I, ¹²⁴I, or ¹³¹I, for example, the noninvasive three-dimensional imaging of pancreatic β-cells by SPECT, PET, or the like is enabled, and preferably, the quantification of pancreatic β-cells is enabled.

### (Example 4)

A molecular probe (SEQ ID NO. 20) of the following formula (20) was prepared, which had a configuration in the amino acid sequence of SEC ID NO. 5, the amino group of the side chain of the lysine residue at position 32 was labeled with 3-[¹²³I]iodobenzoyl group (hereinafter, referred to also as "[¹²³I]IB label"), the carboxyl group at the C-terminus was amidated and the α-amino group at the N-terminus was not acetylated. The molecular probe of the following formula (20) was prepared in the same manner as in Example 3 except that [¹²³I]SIB was used in place of [¹²⁵I]SIB.

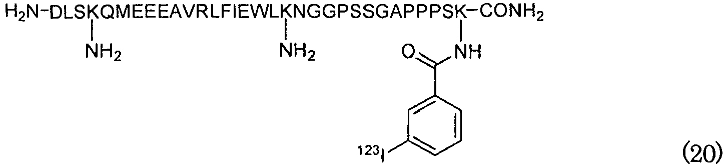

### [Three-dimensional imaging]

Using the molecular probe of the above formula (20), SPECT imaging of mice was carried out. The molecular probe of the above formula (20) (243 µCi/120µL) was administered to anesthetized 6-week-old ddY mice (male, weight: about 30g) by intravenous injection, and a SPECT imaging was carried out. The SPECT imaging was carried out under the following imaging conditions for 32 minutes starting at the point of 30 minutes after the administration of the molecular probe, with use of a gamma camera (product name: SPECT2000H-40 manufactured by Hitachi Medical Corporation). Images obtained were reconfigured under the following reconfiguration conditions.

### Imaging condition

Collimator: LEPH pinhole collimator
Collection angle of detector: 360° at 11.25°/60sec
Collection time: 60 sec × 32 frames, 32 minutes

### Reconfiguration condition

Pretreatment filter: Buttenwor th filter (oder:10, cutoff frequency: 0.13)

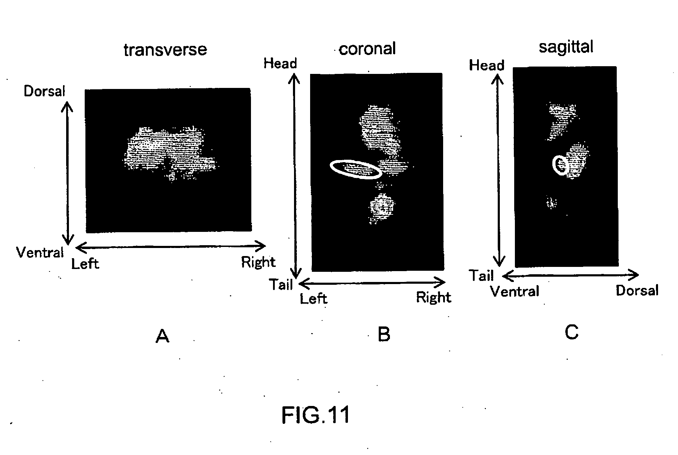

Exemplary results are shown in FIGS. 11A to 11C. The images were taken at 30 minutes after the administration of the molecular probe (accumulation integrating time: 32 minutes). FIG. 11A shows a transverse view, and FIG. 11B shows a coronal view, and FIG. 11C is sagittal view. The white circles in FIGS. 11B and 11C indicate the positions of the pancreas. It should be noted that the images of FIGS. 11A to 11C are at the same contrast.

As shown in FIGS. 11A to 11C, the position of the pancreas was confirmed noninvasively in mice with use of the molecular probe of the above formula (20). In other words, it was confirmed that the molecular probe enables the noninvasive three-dimensional imaging of the pancreatic islet.

Thus, in view of that the position of the pancreas was confirmed noninvasively in a mouse that has the pancreas in a smaller size than that of a human and in which the organs are present more densely than in a human, this suggests that in a human that has the pancreas in a greater size than that of a mouse and in which the organs are present not as densely as in a mouse, the position of the pancreas and the size of the pancreas can be determined more clearly, and moreover, an amount of expression of the probe in the pancreas can be determined. Therefore, it was suggested that the molecular probe for pancreatic islets imaging of the present invention should enable noninvasive three-dimensional imaging of the pancreas in a human, particularly noninvasive three-dimensional imaging of pancreatic β-cells.

### Industrial Applicability

As described above, the present invention is useful in, for example, the medical field, the molecule imaging field, and the field relating to diabetes.

### Sequence Listing Free Text

SEQ ID NOS. 1 to 4: the amino acid sequences of the molecular probe precursors as described herein
SEQ ID NOS. 5 to 8: the amino acid sequences of the molecular probes as described herein
SEQ ID NO. 9: the amino acid sequence of polypeptide used for producing a molecular probe precursor of Example 1
SEQ ID NO. 10: the amino acid sequence of the molecular probe precursor of Example 1
SEQ ID NO. 11: the amino acid sequence of the molecular probe of Example 1
SEQ ID NO. 12: the amino acid sequence of the molecular probe precursor of Reference Example 1
SEQ ID NO. 13: the amino acid sequence of the molecular probe of Reference Example 1
SEQ ID NO. 14: the amino acid sequence of the molecular probe precursor of Reference Example 2
SEQ ID NO. 15: the amino acid sequence of the molecular probe of Reference Example 2
SEQ ID NO. 16: the amino acid sequence of the molecular probe precursor of Example 2
SEQ ID NO. 17: the amino acid sequence of the molecular probe of Example 2
SEQ ID NO. 18: the amino acid sequence of the molecular probe used in the Binding Assay
SEQ ID NO. 19: the amino acid sequence of the molecular probe of Example 3
SEQ ID NO. 20: the amino acid sequence of the molecular probe of Example 4.

## Claims

1. Use of a precursor of a molecular probe for imaging of pancreatic islets, the precursor comprising any one of the following polypeptides:
a polypeptide represented by any one of the following formulae (1) to (4),
*-DLSK* QMEEEAVRLFIEWLK* NGGPSSGAPPPSK-NH₂ (1) (SEQ ID NO. 1)
*-LSK* QMEEEAVRLFIEWLK* NGGPSSGAPPPSK-NH₂ (2) (SEQ ID NO. 2)
*-SK* QMEEEAVRLFIEWLK* NGGPSSGAPPPSK-NH₂ (3) (SEQ ID NO. 3)
*-K* QMEEEAVRLFIEWLK* NGGPSSGAPPPSK-NH₂ (4) (SEQ ID NO. 4)
in the formulae (1) to (4), *- indicates that the α-amino group at the N-terminus is protected by a protecting group or modified with a modifying group having no electric charge, K* indicates that the amino group of the side chain of the lysine is protected by a protecting group, and -NH₂ indicates that the carboxyl group at the C-terminus is amidated, wherein said precursor is used to obtain the molecular probe and said molecular probe binds to pancreatic islets, wherein the amino group of the side chain of the lysine at the C-terminus is labeled with a labeling compound comprising an aromatic ring having a radioactive nuclide.

2. Use according to claim 1, wherein the modifying group having no electric charge is selected from the group consisting of acetyl group, benzyl group, benzyloxymethyl group, o-bromobenzyloxycarbonyl group, t-butyl group, t-butyldimethylsilyl group, 2-chlorobenzyl group, 2,6-dichlorobenzyl group, cyclohexyl group, cyclopentyl group, isopropyl group, pivalyl group, tetrahydropyran-2-yl group, tosyl group, trimethylsilyl group, and trityl group.

3. A method for producing a molecular probe for imaging of pancreatic islets, comprising labeling and deprotecting the precursor of a molecular probe, wherein the precursor comprises any one of the following polypeptides:
a polypeptide represented by any one of the following formulae (1) to (4),
*-DLSK* QMEEEAVRLFIEWLK* NGGPSSGAPPPSK-NH₂ (1) (SEQ ID NO. 1)
*-LSK* QMEEEAVRLFIEWLK* NGGPSSGAPPPSK-NH₂ (2) (SEQ ID NO. 2)
*-SK* QMEEEAVRLFIEWLK* NGGPSSGAPPPSK-NH₂ (3) (SEQ ID NO. 3)
*-K* QMEEEAVRLFIEWLK* NGGPSSGAPPPSK-NH₂ (4) (SEQ ID NO. 4)
in the formulae (1) to (4), *- indicates that the α-amino group at the N-terminus is protected by a protecting group or modified with a modifying group having no electric charge, K* indicates that the amino group of the side chain of the lysine is protected by a protecting group, and -NH₂ indicates that the carboxyl group at the C-terminus is amidated, and
wherein the labeling of the precursor of the molecular probe comprises labeling of the amino group of the side chain of the lysine at the C-terminus with a labeling compound comprising an aromatic ring having a radioactive nuclide.

4. The method according to claim 3, wherein the modifying group having no electric charge is selected from the group consisting of acetyl group, benzyl group, benzyloxymethyl group, o-bromobenzyloxycarbonyl group, t-butyl group, t-butyldimethylsilyl group, 2-chlorobenzyl group, 2,6-dichlorobenzyl group, cyclohexyl group, cyclopentyl group, isopropyl group, pivalyl group, tetrahydropyran-2-yl group, tosyl group, trimethylsilyl group, and trityl group.

5. The method according to claim 3 or 4, wherein the labeling compound comprising the aromatic ring comprises a group represented by chemical formula (I) below, 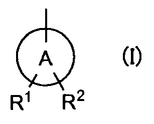 in the formula (I), A represents any of an aromatic hydrocarbon group and an aromatic heterocycle; R¹ represents a substituent comprising ¹⁸F, ⁷⁵Br, ⁷⁶Br, ⁷⁷Br, ¹²³I, ¹²⁴I, ¹²⁵I, or ¹³¹I; and R² represents a hydrogen atom or one or more substituents different from the substituents represented by R¹.

6. A molecular probe for imaging of pancreatic islets, comprising any one of the following polypeptides:
a polypeptide represented by any one of the following formulae (5) to (8),
Z-DLSKQMEEEAVRLFIEWLKNGGPSSGAPPPSX-NH₂ (5) (SEQ ID NO. 5)
Z-LSKQMEEEAVRLFIEWLKNGGPSSGAPPPSX-NH₂ (6) (SEQ ID NO. 6)
Z-SKQMEEEAVRLFIEWLKNGGPSSGAPPPSX-NH₂ (7) (SEQ ID NO. 7)
Z-KQMEEEAVRLFIEWLKNGGPSSGAPPPSX-NH₂ (8) (SEQ ID NO. 8)
in the formulae (5) to (8), X represents a lysine residue having the amino group of the side chain labeled with a radioactive nuclide, where said amino group of the side chain of the lysine is bonded to a group comprising an aromatic ring represented by chemical formula (III) below, 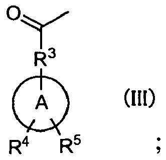 Z- indicates that the α-amino group at the N-terminus is unmodified or modified with a modifying group having no electric charge; and, -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group.

7. Use of a molecular probe for imaging of pancreatic islets, comprising any one of the following polypeptides:
a polypeptide represented by any one of the following formulae (5) to (8),
Z-DLSKQMEEEAVRLFIEWLKNGGPSSGAPPPSX-NH₂ (5) (SEQ ID NO. 5)
Z-LSKQMEEEAVRLFIEWLKNGGPSSGAPPPSX-NH₂ (6) (SEQ ID NO. 6)
Z-SKQMEEEAVRLFIEWLKNGGPSSGAPPPSX-NH₂ (7) (SEQ ID NO. 7)
Z-KQMEEEAVRLFIEWLKNGGPSSGAPPPSX-NH₂ (8) (SEQ ID NO. 8)
in the formulae (5) to (8), X represents a lysine residue having the amino group of the side chain labeled with a radioactive nuclide, where said amino group of the side chain of the lysine is bonded to a group comprising an aromatic ring represented by chemical formula (III) below, 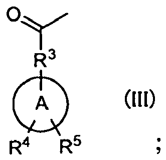 Z- indicates that the α-amino group at the N-terminus is unmodified or modified with a modifying group having no electric charge; and, -NH₂ indicates that the carboxyl group at the C-terminus is amidated, and in the formula (III), A represents any of an aromatic hydrocarbon group or an aromatic heterocyclic group; R⁴ represents a substituent comprising ¹⁸F, ⁷⁵Br, ⁷⁶Br, ⁷⁷Br, ¹²³I, ¹²⁴I, ¹²⁵I, or ¹³¹I; R⁵ indicates a hydrogen atom or one or more substituents different from the substituents represented by R⁴; and R³ represents any of a bond, a C₁-C₆ alkylene group and a C₁-C₆ oxyalkylene group.

8. A kit for preparing a molecular probe for imaging of pancreatic islets, comprising the precursor of a molecular probe as defined in claim 3 or 4, wherein the kit further comprises a compound for labeling the precursor of a molecular probe for imaging of pancreatic islets, wherein the compound comprises an aromatic ring having a radioactive nuclide.

9. The kit according to claim 8, wherein the compound comprising the aromatic ring is a compound having a group represented by chemical formula (I) below, 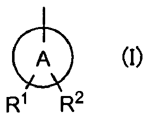 in the formula (I), A represents any of an aromatic hydrocarbon group or an aromatic heterocycle, R¹ represents a substituent comprising ¹⁸F, ⁷⁵Br, ⁷⁶Br, ⁷⁷Br, ¹²³I, ¹²⁴I, ¹²⁵I, or ¹³¹I, and R² represents a hydrogen atom or one or more substituents different from the substituents represented by R¹.

10. A kit for performing pancreatic islets imaging, comprising the molecular probe for imaging of pancreatic islets as defined in claim 6 or 7.

11. A method for imaging of pancreatic islets comprising:
(A) labeling and deprotecting the precursor of a molecular probe as defined in claim 3 or 4, wherein the labeling of the precursor comprises labeling the amino group of the side chain of the lysine at the C-terminus with a labeling compound comprising an aromatic ring having a radioactive nuclide, and imaging pancreatic islets by using the labeled and deprotected molecular probe for imaging of pancreatic islets, or
(B) detecting a signal of the molecular probe as defined in claim 6 or 7 from an analyte to which the probe has been administered.

12. A method for determining the amount of pancreatic islets, comprising:
(A) preparing a molecular probe for imaging of pancreatic islets by labeling and deprotecting the precursor of the molecular probe as defined in claim 3 or 4, wherein the labeling of the precursor comprises labeling the amino group of the side chain of the lysine at the C-terminus with a labeling compound comprising an aromatic ring having a radioactive nuclide; and
calculating the amount of pancreatic islets based on the results of imaging of pancreatic islets with use of the molecular probe, or
(B) detecting a signal of the molecular probe as defined in claim 6 or 7 for imaging of pancreatic islets from an analyte to which the probe has been administered; and calculating the amount of pancreatic islets based on the detected signal of the molecular probe for imaging of pancreatic islets.

13. The molecular probe as defined in claim 6 or 7 for use in prevention, treatment, or *in vivo* diagnosis of diabetes, wherein in said prevention, treatment, or diagnosis said molecular probe is used in the imaging of pancreatic islets.

## Patentansprüche

1. Verwendung eines Vorläufers einer molekularen Sonde zur Bildgebung von Langerhans-Inseln (Pankreasinseln), wobei der Vorläufer irgendeines der folgenden Polypeptide umfasst:
ein Polypeptid, das durch irgendeine der folgenden Formeln (1) bis (4) dargestellt wird:
*-DLSK* QMEEEAVRLFIEWLK* NGGPSSGAPPPSK-NH₂ (1) (SEQ ID NO.1),
*-LSK* QMEEEAVRLFIEWLK* NGGPSSGAPPPSK-NH₂ (2) (SEQ ID NO.2),
*-SK* QMEEEAVRLFIEWLK* NGGPSSGAPPPSK-NH₂ (3) (SEQ ID NO.3),
*-K* QMEEEAVRLFIEWLK* NGGPSSGAPPPSK-NH₂ (4) (SEQ ID NO.4),
wobei in den Formeln (1) bis (4) *- angibt, dass die α-Aminogruppe an dem N-Terminus durch eine Schutzgruppe geschützt ist oder durch eine modifizierende Gruppe, die keine elektrische Ladung aufweist, modifiziert wird, wobei K* angibt, dass die Aminogruppe der Seitenkette des Lysins durch eine Schutzgruppe geschützt ist, und wobei -NH₂ angibt, dass die Carboxylgruppe an dem C-Terminus amidiert ist, wobei der Vorläufer verwendet wird, um die molekulare Sonde zu erhalten, und wobei die molekulare Sonde an die Langerhans-Inseln bindet, wobei die Aminogruppe der Seitenkette des Lysins an dem C-Terminus mit einer Markierungsverbindung, die einen aromatischen Ring mit einem radioaktiven Nuklid umfasst, markiert ist.

2. Verwendung nach Anspruch 1, wobei die modifizierende Gruppe, die keine elektrische Ladung aufweist, ausgewählt ist aus der Gruppe, die aus der Acetylgruppe, der Benzylgruppe, der Benzyloxymethylgruppe, der o-Bromobenzyloxycarbonylgruppe, der t-Butylgruppe, der der t-Butyldimethylsilylgruppe, 2-Chlorobenzylgruppe, 2,6-Dichlorobenzylgruppe, Cyclohexylgruppe, Cyclopentylgruppe, Isopropylgruppe, Pivalylgruppe, Tetrahydropropan-2-yl-gruppe, Tosylgruppe, Trimethylsilylgruppe und aus der Tritylgruppe besteht.

3. Verfahren zum Herstellen einer molekularen Sonde zur Bildgebung von Langerhans-Inseln, das ein Markieren und ein Entschützen des Vorläufers einer molekularen Sonde umfasst, wobei der Vorläufer irgendeines der folgenden Polypeptide umfasst:
ein Polypeptid, das durch irgendeine der folgenden Formeln (1) bis (4) dargestellt wird:
*-DLSK* QMEEEAVRLFIEWLK* NGGPSSGAPPPSK-NH₂ (1) (SEQ ID NO.1),
*-LSK* QMEEEAVRLFIEWLK* NGGPSSGAPPPSK-NH₂ (2) (SEQ ID NO.2),
*-SK* QMEEEAVRLFIEWLK* NGGPSSGAPPPSK-NH₂ (3) (SEQ ID NO.3),
*-K* QMEEEAVRLFIEWLK* NGGPSSGAPPPSK-NH₂ (4) (SEQ ID NO.4),
wobei in den Formeln (1) bis (4) *- angibt, dass die α-Aminogruppe an dem N-Terminus durch eine Schutzgruppe geschützt ist oder durch eine modifizierende Gruppe, die keine elektrische Ladung aufweist, modifiziert wird, wobei K* angibt, dass die Aminogruppe der Seitenkette des Lysins durch eine Schutzgruppe geschützt ist, und wobei -NH₂ angibt, dass die Carboxylgruppe an dem C-Terminus amidiert ist, und
wobei das Markieren des Vorläufers der molekularen Sonde ein Markieren der Aminogruppe der Seitenkette des Lysins an dem C-Terminus mit einer Markierungsverbindung umfasst, die einen aromatischen Ring mit einem radioaktiven Nuklid umfasst.

4. Verfahren nach Anspruch 3, wobei die modifizierende Gruppe, die keine elektrische Ladung aufweist, ausgewählt ist aus der Gruppe, die aus der Acetylgruppe, der Benzylgruppe, der Benzyloxymethylgruppe, der o-Bromobenzyloxycarbonylgruppe, der t-Butylgruppe, der der t-Butyldimethylsilylgruppe, 2-Chlorobenzylgruppe, 2,6-Dichlorobenzylgruppe, Cyclohexylgruppe, Cyclopentylgruppe, Isopropylgruppe, Pivalylgruppe, Tetrahydropropan-2-yl-gruppe, Tosylgruppe, Trimethylsilylgruppe und aus der Tritylgruppe besteht.

5. Verfahren nach Anspruch 3 oder 4, wobei die Markierungsverbindung, die den aromatischen Ring umfasst, eine Gruppe umfasst, die durch die folgende chemische Formel (I) dargestellt ist, 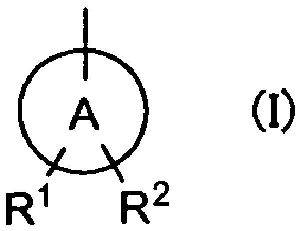 wobei in der Formel (I), A irgendeine aromatische Kohlenwasserstoffgruppe und einen aromatischen Heterozyklus darstellt; R¹ einen Substituenten darstellt, der ¹⁸F, ⁷⁵Br, ⁷⁶Br, ⁷⁷Br, ¹²³I, ¹²⁴I, ¹²⁵I oder ¹³¹I umfasst; und R² ein Wasserstoffatom oder einen oder mehrere Substituenten darstellt, die andere als diejenigen sind, die von R¹ dargestellt werden.

6. Molekulare Sonde zur Bildgebung von Langerhans-Inseln, die irgendeines der folgenden Polypeptide umfasst:
ein Polypeptid, das durch irgendeine der folgenden Formeln (5) bis (8) dargestellt wird:
Z-DLSKQMEEEAVRLFIEWLKNGGPSSGAPPPSX-NH₂ (5) (SEQ ID NO.5),
Z-LSKQMEEEAVRLFIEWLKNGGPSSGAPPPSX-NH₂ (6) (SEQ ID NO.6),
Z-SKQMEEEAVRLFIEWLKNGGPSSGAPPPSX-NH₂ (7) (SEQ ID NO.7),
Z-KQMEEEAVRLFIEWLKNGGPSSGAPPPSX-NH₂ (8) (SEQ ID NO.8),
wobei in den Formeln (5) bis (8) X einen Lysinrest darstellt, der die Aminogruppe der Seitenkette aufweist, die mit einem radioaktiven Nuklid markiert ist, wobei die Aminogruppe der Seitenkette des Lysins an eine Gruppe gebunden ist, die einen aromatischen Ring umfasst und durch die folgende chemische Formel (III) dargestellt ist, 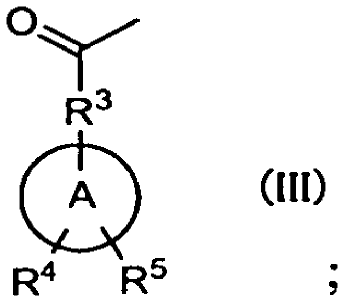 wobei Z- angibt, dass die α-Aminogruppe an dem N-Terminus nicht modifiziert oder mit einer modifizierenden Gruppe, die keine elektrische Ladung aufweist, modifiziert ist; und wobei -NH₂ angibt, dass die Carboxylgruppe an dem C-Terminus amidiert ist, und wobei in der Formel (III), (A) irgendeine aromatische Kohlenwasserstoffgruppe oder eine aromatische Heterozyklusgruppe darstellt; R⁴ einen Substituenten darstellt, der ¹⁸F, ⁷⁵Br, ⁷⁶Br, ⁷⁷Br, ¹²³I, ¹²⁴I, ¹²⁵I oder ¹³¹I umfasst; wobei R⁵ ein Wasserstoffatom oder einen oder mehrere Substituenten angibt, die andere als diejenigen sind, die von R⁴ dargestellt werden; und R³ irgendeine Bindung, eine C₁-C₆-Alkylengruppe und eine C₁-C₆-Oxyalkylengruppe darstellt.

7. Verwendung einer molekularen Sonde zur Bildgebung von Langerhans-Inseln, die irgendeines der folgenden Polypeptide umfasst:
ein Polypeptid, das durch irgendeine der folgenden Formeln (5) bis (8) dargestellt wird:
Z-DLSKQMEEEAVRLFIEWLKNGGPSSGAPPPSX-NH₂ (5) (SEQ ID NO.5),
Z-LSKQMEEEAVRLFIEWLKNGGPSSGAPPPSX-NH₂ (6) (SEQ ID NO.6),
Z-SKQMEEEAVRLFIEWLKNGGPSSGAPPPSX-NH₂ (7) (SEQ ID NO.7),
Z-KQMEEEAVRLFIEWLKNGGPSSGAPPPSX-NH₂ (8) (SEQ ID NO.8),
wobei in den Formeln (5) bis (8) X einen Lysinrest darstellt, der die Aminogruppe der Seitenkette aufweist, die mit einem radioaktiven Nuklid markiert ist, wobei die Aminogruppe der Seitenkette des Lysins an eine Gruppe gebunden ist, die einen aromatischen Ring umfasst und durch die folgende chemische Formel (III) dargestellt ist, 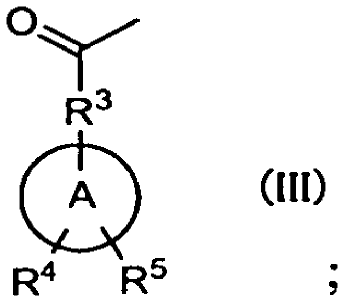 wobei Z- angibt, dass die α-Aminogruppe an dem N-Terminus nicht modifiziert oder mit einer modifizierenden Gruppe, die keine elektrische Ladung aufweist, modifiziert ist; und wobei -NH₂ angibt, dass die Carboxylgruppe an dem C-Terminus amidiert ist, und wobei in der Formel (III), A irgendeine aromatische Kohlenwasserstoffgruppe oder eine aromatische Heterozyklusgruppe darstellt; R⁴ einen Substituenten darstellt, der ¹⁸F, ⁷⁵Br, ⁷⁶Br, ⁷⁷Br, ¹²³I, ¹²⁴I, ¹²⁵I oder ¹³¹I umfasst; wobei R⁵ ein Wasserstoffatom oder einen oder mehrere Substituenten angibt, die andere als diejenigen sind, die von R⁴ dargestellt werden; und R³ irgendeine Bindung, eine C₁-C₆-Alkylengruppe und eine C₁-C₆-Oxyalkylengruppe darstellt.

8. Ausrüstungssatz (Kit) zum Herstellen einer molekularen Sonde zur Bildgebung von Langerhans-Inseln, der den Vorläufer einer molekularen Sonde, wie nach Anspruch 3 oder 4 definiert, umfasst, wobei der Ausrüstungssatz ferner eine Verbindung zum Markieren des Vorläufers einer molekularen Sonde zur Bildgebung von Langerhans-Inseln umfasst, wobei die Verbindung einen aromatischen Ring mit einem radioaktiven Nuklid umfasst.

9. Ausrüstungssatz nach Anspruch 8, wobei die Verbindung, die den aromatischen Ring umfasst, eine Verbindung ist, die eine Gruppe aufweist, die durch die folgende chemische Formel (I) dargestellt ist, 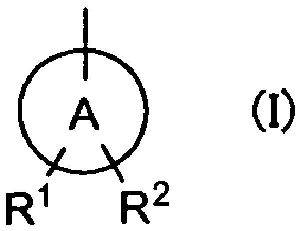 wobei in der Formel (I), A irgendeine aromatische Kohlenwasserstoffgruppe oder einen aromatischen Heterozyklus darstellt; R¹ einen Substituenten darstellt, der ¹⁸F, ⁷⁵Br, ⁷⁶Br, ⁷⁷Br, ¹²³I, ¹²⁴I, ¹²⁵I oder ¹³¹I umfasst; und R² ein Wasserstoffatom oder einen oder mehrere Substituenten darstellt, die andere als diejenigen sind, die von R¹ dargestellt werden.

10. Ausrüstungssatz zum Durchführen der Bildgebung von Langerhans-Inseln, der die molekulare Sonde zur Bildgebung von Langerhans-Inseln umfasst, so wie nach Anspruch 6 oder 7 definiert.

11. Verfahren zur Bildgebung von Langerhans-Inseln, das umfasst:
(A) Markieren und Entschützen des Vorläufers einer molekularen Sonde, so wie nach Anspruch 3 oder 4 definiert, wobei das Markieren des Vorläufers ein Markieren der Aminogruppe der Seitenkette des Lysins an dem C-Terminus mit einer Markierungsverbindung, die einen aromatischen Ring mit einem radioaktiven Nuklid umfasst, und ein Abbilden von Langerhans-Inseln unter Verwendung der markierten und entschützten molekularen Sonde zur Bildgebung von Langerhans-Inseln umfasst, oder
(B) Detektieren eines Signals der molekularen Sonde, so wie nach Anspruch 6 oder 7 definiert, von einem Analyten, an dem die Sonde appliziert wird.

12. Verfahren zum Bestimmen der Menge an Langerhans-Inseln, das umfasst:
(A) Herstellen einer molekularen Sonde zur Bildgebung von Langerhans-Inseln durch Markieren und Entschützen des Vorläufers der molekularen Sonde, so wie nach Anspruch 3 oder 4 definiert, wobei das Markieren des Vorläufers ein Markieren der Aminogruppe der Seitenkette des Lysins an dem C-Terminus mit einer Markierungsverbindung umfasst, die einen aromatischen Ring mit einem radioaktiven Nuklid umfasst; und
Berechnen der Menge an Langerhans-Inseln auf der Grundlage der Ergebnisse der Bildgebung von Langerhans-Inseln mit der Verwendung der molekularen Sonde, oder
(B) Detektieren eines Signals der molekularen Sonde, so wie nach Anspruch 6 oder 7 definiert, zur Bildgebung von Langerhans-Inseln von einem Analyten, an dem die Sonde appliziert wird; und
Berechnen der Menge an Langerhans-Inseln auf der Grundlage des detektierten Signals der molekularen Sonde zur Bildgebung von Langerhans-Inseln.

13. Molekulare Sonde nach Anspruch 6 oder 7 für eine Verwendung in der Prävention, bei der Behandlung oder bei der *in vivo* Diagnose von Diabetes, wobei in der Prävention, bei der Behandlung oder bei der Diagnose die molekulare Sonde zur Bildgebung von Langerhans-Inseln verwendet wird.

## Revendications

1. Utilisation d'un précurseur d'une sonde moléculaire pour l'imagerie d'îlots pancréatiques, le précurseur comprenant n'importe lequel des polypeptides suivants :
un polypeptide représenté par n'importe laquelle des formules suivantes (1) à (4),
*-DLSK* QMEEEAVRLFIEWLK* NGGPSSGAPPPSK-NH₂ (1) (SEQ ID n° 1)
*-LSK* QMEEEAVRLFIEWLK* NGGPSSGAPPPSK-NH₂ (2) (SEQ ID n° 2)
*-SK* QMEEEAVRLFIEWLK* NGGPSSGAPPPSK-NH₂ (3) (SEQ ID n° 3)
*-K* QMEEEAVRLFIEWLK* NGGPSSGAPPPSK-NH₂ (4) (SEQ ID n° 4)
dans les formules (1) à (4), *- indique que le groupe α-amino au niveau de la terminaison N est protégé par un groupe de protection ou modifié avec un groupe de modification n'ayant aucune charge électrique, K* indique que le groupe amino de la chaîne latérale de la lysine est protégé par un groupe de protection, et -NH₂ indique que le groupe carboxyle au niveau de la terminaison C est amidé, dans laquelle ledit précurseur est utilisé pour obtenir la sonde moléculaire et ladite sonde moléculaire se lie aux îlots pancréatiques, dans laquelle le groupe amino de la chaîne latérale de la lysine au niveau de la terminaison C est étiqueté avec un composé d'étiquetage comprenant un noyau aromatique ayant un nucléide radioactif.

2. Utilisation selon la revendication 1, dans laquelle le groupe de modification n'ayant aucune charge électrique est sélectionné à partir du groupe constitué par un groupe acétyle, un groupe benzyle, un groupe benzyloxyméthyle, un groupe o-bromobenzyloxycarbonyle, un groupe t-butyle, un groupe t-butyldiméthylsilyle, un groupe 2-chlorobenzyle, un groupe 2,6-dichlorobenzyle, un groupe cyclohexyle, un groupe cyclopentyle, un groupe isopropyle, un groupe pivalyle, un groupe tétrahydropyran-2-yle, un groupe tosyle, un groupe triméthylsilyle et un groupe trityle.

3. Procédé pour produire une sonde moléculaire pour l'imagerie d'îlots pancréatiques, comprenant l'étiquetage et la déprotection du précurseur d'une sonde moléculaire, dans lequel le précurseur comprend n'importe lequel des polypeptides suivants :
un polypeptide représenté par n'importe laquelle des formules suivantes (1) à (4),
*-DLSK* QMEEEAVRLFIEWLK* NGGPSSGAPPPSK-NH₂ (1) (SEQ ID n° 1)
*-LSK* QMEEEAVRLFIEWLK* NGGPSSGAPPPSK-NH₂ (2) (SEQ ID n° 2)
*-SK* QMEEEAVRLFIEWLK* NGGPSSGAPPPSK-NH₂ (3) (SEQ ID n° 3)
*-K* QMEEEAVRLFIEWLK* NGGPSSGAPPPSK-NH₂ (4) (SEQ ID n° 4)
dans les formules (1) à (4), *- indique qu'un groupe amino au niveau de la terminaison N est protégé par un groupe de protection ou modifié avec un groupe de modification n'ayant aucune charge électrique, K* indique que le groupe amino de la chaîne latérale de la lysine est protégé par un groupe de protection, et -NH₂ indique que le groupe carboxyle au niveau de la terminaison C est amidé, et
dans lequel l'étiquetage du précurseur de la sonde moléculaire comprend l'étiquetage du groupe amino de la chaîne latérale de la lysine au niveau de la terminaison C avec un composé d'étiquetage comprenant un noyau aromatique ayant un nucléide radioactif.

4. Procédé selon la revendication 3, dans lequel le groupe de modification n'ayant aucune charge électrique est sélectionné à partir du groupe constitué par un groupe acétyle, un groupe benzyle, un groupe benzyloxyméthyle, un groupe o-bromobenzyloxycarbonyle, un groupe t-butyle, un groupe t-butyldiméthylsilyle, un groupe 2-chlorobenzyle, un groupe 2,6-dichlorobenzyle, un groupe cyclohexyle, un groupe cyclopentyle, un groupe isopropyle, un groupe pivalyle, un groupe tétrahydropyran-2-yle, un groupe tosyle, un groupe triméthylsilyle et un groupe trityle.

5. Procédé selon la revendication 3 ou 4, dans lequel le composé d'étiquetage comprenant le noyau aromatique comprend un groupe représenté par la formule chimique (I) ci-dessous, 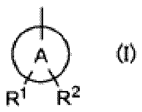 dans la formule (I), A représente n'importe lequel d'un groupe hydrocarbure aromatique et d'un composé hétérocyclique aromatique ; R¹ représente un substituant comprenant ¹⁸F, ⁷⁵Br, ⁷⁶Br, ⁷⁷Br, ¹²⁸I, ¹²⁴I, ¹²⁵I, ou ¹³¹I ; et R² représente un atome d'hydrogène ou un ou plusieurs substituants différents des substituants représentés par R¹.

6. Sonde moléculaire pour l'imagerie d'îlots pancréatiques, comprenant n'importe lequel des polypeptides suivants :
un polypeptide représenté par n'importe laquelle des formules suivantes (5) à (8),
Z-DLSKQMEEEAVRLFIEWLKNGGPSSGAPPPSX-NH₂ (5) (SEQ ID n° 5)
Z-LSKQMEEEAVRLFIEWLKNGGPSSGAPPPSX-NH₂ (6) (SEQ ID n° 6)
Z-SKQMEEEAVRLFIEWLKNGGPSSGAPPPSX-NH₂ (7) (SEQ ID n° 7)
Z-KQMEEEAVRLFIEWLKNGGPSSGAPPPSX-NH₂ (8) (SEQ ID n° 8)
dans les formules (5) à (8), X représente un résidu de lysine ayant le groupe amino de la chaîne latérale étiqueté avec un nucléide radioactif, où ledit groupe amino de la chaîne latérale de la lysine est lié à un groupe comprenant un noyau aromatique représenté par la formule chimique (III) ci-dessous, 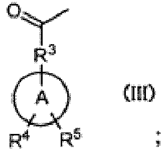 Z⁻ indique que le groupe α-amino au niveau de la terminaison N est inchangé ou modifié avec un groupe de modification n'ayant aucune charge électrique ; et,-NH₂ indique que le groupe carboxyle au niveau de la terminaison C est amidé, et dans la formule (III), A représente n'importe lequel d'un groupe hydrocarbure aromatique ou d'un groupe hétérocyclique aromatique ; R⁴ représente un substituant comprenant ¹⁸F, ⁷⁵Br, ⁷⁶Br, ⁷⁷Br, ¹²³I, ¹²⁴I, ¹²⁵I, ou ¹³¹I ; R⁵ indique un atome d'hydrogène ou un ou plusieurs substituants différents des substituants représentés par R⁴ ; et R³ représente n'importe laquelle d'une liaison, d'un groupe alkylène en C₁-C₆ et d'un groupe oxyalkylène en C₁-C₆.

7. Utilisation d'une sonde moléculaire pour l'imagerie d'îlots pancréatiques, comprenant n'importe lequel des polypeptides suivants :
un polypeptide représenté par n'importe laquelle des formules suivantes (5) à (8),
Z-DLSKQMEEEAVRLFIEWLKNGGPSSGAPPPSX-NH₂ (5) (SEQ ID n° 5)
Z-LSKQMEEEAVRLFIEWLKNGGPSSGAPPPSX-NH₂ (6) (SEQ ID n° 6)
Z-SKQMEEEAVRLFIEWLKNGGPSSGAPPPSX-NH₂ (7) (SEQ ID n° 7)
Z-KQMEEEAVRLFIEWLKNGGPSSGAPPPSX-NH₂ (8) (SEQ ID n° 8)
dans les formules (5) à (8), X représente un résidu de lysine ayant le groupe amino de la chaîne latérale étiqueté d'un nucléide radioactif, où ledit groupe amino de la chaîne latérale de la lysine est lié à un groupe comprenant un noyau aromatique représenté par la formule chimique (III) ci-dessous, 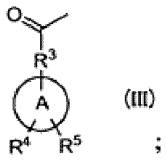 Z⁻ indique que le groupe α-amino au niveau de la terminaison N est inchangé ou modifié avec un groupe de modification n'ayant aucune charge électrique ; et,-NH₂ indique que le groupe carboxyle au niveau de la terminaison C est amidé, et dans la formule (III), A représente n'importe lequel d'un groupe hydrocarbure aromatique ou d'un groupe hétérocyclique aromatique ; R⁴ représente un substituant comprenant ¹⁸F, ⁷⁵Br, ⁷⁶Br, ⁷⁷Br, ¹²³I, ¹²⁴I, ¹²⁵I, ou ¹³¹I ; R⁵ indique un atome d'hydrogène ou un ou plusieurs substituants différents des substituants représentés par R⁴ ; et R³ représente n'importe laquelle d'une liaison, d'un groupe alkylène en C₁-C₆ et d'un groupe oxyalkylène en C₁-C₆.

8. Kit pour préparer une sonde moléculaire pour l'imagerie d'îlots pancréatiques, comprenant le précurseur d'une sonde moléculaire telle que défini dans la revendication 3 ou 4, dans lequel le kit comprend en outre un composé pour étiqueter le précurseur d'une sonde moléculaire pour l'imagerie d'îlots pancréatiques, dans lequel le composé comprend un noyau aromatique ayant un nucléide radioactif.

9. Kit selon la revendication 8, dans lequel le composé comprenant le noyau aromatique est un composé ayant un groupe représenté par la formule chimique (I) ci-dessous, 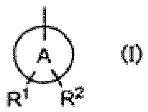 dans la formule (I), A représente n'importe lequel d'un groupe hydrocarbure aromatique ou d'un composé hétérocyclique aromatique, R¹ représente un substituant comprenant ¹⁸F, ⁷⁵Br, ⁷⁶Br, ⁷⁷Br, ¹²³I, ¹²⁴I, ¹²⁵I, ou ¹³¹I, et R² représente un atome d'hydrogène ou un ou plusieurs substituants différents des substituants représentés par R¹.

10. Kit pour effectuer l'imagerie d'îlots pancréatique, comprenant la sonde moléculaire pour l'imagerie d'îlots pancréatiques telle que définie dans la revendication 6 ou 7.

11. Procédé pour l'imagerie d'îlots pancréatiques comprenant :
(A) l'étiquetage et la déprotection du précurseur d'une sonde moléculaire telle que défini dans la revendication 3 ou 4, dans lequel l'étiquetage du précurseur comprend l'étiquetage du groupe amino de la chaîne latérale de la lysine au niveau de la terminaison C avec un composé d'étiquetage comprenant un noyau aromatique ayant un nucléide radioactif, et l'imagerie d'îlots pancréatiques en utilisant la sonde moléculaire étiquetée et déprotégée pour l'imagerie d'îlots pancréatiques, ou
(B) la détection d'un signal de la sonde moléculaire telle que définie dans la revendication 6 ou 7 à partir d'une substance à analyser à laquelle la sonde a été administrée.

12. Procédé pour déterminer la quantité d'îlots pancréatiques, comprenant :
(A) la préparation d'une sonde moléculaire pour l'imagerie d'îlots pancréatiques en étiquetant et en déprotégeant le précurseur de la sonde moléculaire tel que défini dans la revendication 3 ou 4, dans lequel l'étiquetage du précurseur comprend l'étiquetage du groupe amino de la chaîne latérale de la lysine au niveau de la terminaison C avec un composé d'étiquetage comprenant un noyau aromatique ayant un nucléide radioactif ; et
le calcul de la quantité d'îlots pancréatiques sur la base des résultats d'imagerie d'îlots pancréatiques avec utilisation de la sonde moléculaire, ou
(B) la détection d'un signal de la sonde moléculaire telle que définie dans la revendication 6 ou 7 pour l'imagerie d'îlots pancréatiques d'une substance à analyser à laquelle la sonde a été administrée; et le calcul de la quantité d'îlots pancréatiques sur la base du signal détecté de la sonde moléculaire pour l'imagerie d'îlots pancréatiques.

13. Sonde moléculaire selon la revendication 6 ou 7 pour une utilisation dans la prévention, le traitement, ou le diagnostic *in vivo* de diabètes, dans laquelle dans lesdits traitement, prévention, ou diagnostic, ladite sonde moléculaire est utilisée dans l'imagerie d'îlots pancréatiques.


### SEQUENCE LISTING

<110> Kyoto University **ARKRAY**, Inc.
<120> MOLECULAR PROBE FOR IMAGING OF PANCREATIC ISLET AND PRECURSOR THEREOF, AND USE OF THE MOLECULAR PROBE OR THE PRESURSOR
<130> 42.13.109723
<140> 10808203.3 <141> 2010-08-09
<150> JP2009-185470
   <151> 2009-08-10
<150> US61/282619
   <151> 2009-08-10
<150> JP2009-272445
   <151> 2009-11-30
<150> JP2010-068257
   <151> 2010-03-24
<150> US61/282741
   <151> 2010-03-25
<160> 20
<170> PatentIn version 3.5
<210> 1
   <211> 32
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a protecting group or is modified by a modified group.
<220>
   <221> MOD_RES
   <222> (4)..(4)
   <223> An amino group of a side chain is protected by a protecting group.
<220>
   <221> MOD_RES
   <222> (19)..(19)
   <223> An amino group of a side chain is protected by a protecting group.
<220>
   <221> MOD_RES
   <222> (32)..(32)
   <223> A carboxyl group of a C-terminus is amidated.
<400> 1
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser Lys
   20 25 30
<210> 2
   <211> 31
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a protecting group or is modified by a modified group.
<220>
   <221> MOD_RES
   <222> (3)..(3)
   <223> An amino group of a side chain is protected by a protecting group.
<220>
   <221> MOD_RES
   <222> (18)..(18)
   <223> An amino group of a side chain is protected by a protecting group.
<220>
   <221> MOD_RES
   <222> (31)..(31)
   <223> A carboxyl group of a C-terminus is amidated.
<400> 2
   Leu Ser Lys Gln Met Glu Glu Glu Ala Val Arg Leu Phe Ile Glu Trp
   1 5 10 15
   Leu Lys Asn Gly Gly Pro Ser Ser Gly Ala Pro Pro Pro Ser Lys
   20 25 30
<210> 3
   <211> 30
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a protecting group or is modified by a modified group.
<220>
   <221> MOD_RES
   <222> (2)..(2)
   <223> An amino group of a side chain is protected by a protecting group.
<220>
   <221> MOD_RES
   <222> (17)..(17)
   <223> An amino group of a side chain is protected by a protecting group.
<220>
   <221> MOD_RES
   <222> (30)..(30)
   <223> A carboxyl group of a C-terminus is amidated.
<400> 3
   Ser Lys Gln Met Glu Glu Glu Ala Val Arg Leu Phe Ile Glu Trp Leu
   1               5                   10                  15
   Lys Asn Gly Gly Pro Ser Ser Gly Ala Pro Pro Pro Ser Lys
               20                  25                  30
<210> 4
   <211> 29
   <212> PRT
   <213> Artificial Sequence
<220>
   <223> A precursor of imaging probe for pancreatic islets
<220>
   <221> MOD_RES
   <222> (1)..(1)
   <223> An alpha-amino group of an N-terminus is protected by a protecting group or is modified by a modified group. An amino group of a side chain is protected by a protecting group.
<220>
   <221> MOD_RES
   <222> (16)..(16)
   <223> An amino group of a side chain is protected by a protecting group.
<220>
   <221> MOD_RES
   <222> (29)..(29)
   <223> A carboxyl group of a C-terminus is amidated.
<400> 4
   Lys Gln Met Glu Glu Glu Ala Val Arg Leu Phe Ile Glu Trp Leu Lys
   1               5                   10                  15
   Asn Gly Gly Pro Ser Ser Gly Ala Pro Pro Pro Ser Lys
               20                  25
<210> 5
   <211> 32
   <212> PRT
   <213> Artificial Sequence
<220>
   <223> An imaging probe for pancreatic islets
<220>
   <221> MOD_RES
   <222> (1)..(1)
   <223> An amino group of an N-terminus is not modified or is modified by a modified group.
<220>
   <221> MOD_RES
   <222> (32)..(32)
   <223> An amino group of a side chain is labeled. A carboxyl group of a C-terminus is amidated.
<400> 5
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 6
   <211> 31
   <212> PRT
   <213> Artificial Sequence
<220>
   <223> An imaging probe for pancreatic islets
<220>
   <221> MOD_RES
   <222> (1)..(1)
   <223> An amino group of an N-terminus is not modified or is modified by a modified group.
<220>
   <221> MOD_RES
   <222> (31)..(31)
   <223> An amino group of a side chain is labeled. A carboxyl group of a C-terminus is amidated.
<400> 6
   Leu Ser Lys Gln Met Glu Glu Glu Ala Val Arg Leu Phe Ile Glu Trp
   1               5                   10                  15
   Leu Lys Asn Gly Gly Pro Ser Ser Gly Ala Pro Pro Pro Ser Lys
               20                  25                  30
<210> 7
   <211> 30
   <212> PRT
   <213> Artificial Sequence
<220>
   <223> An imaging probe for pancreatic islets
<220>
   <221> MOD_RES
   <222> (1)..(1)
   <223> An amino group of an N-terminus is not modified or is modified by a modified group.
<220>
   <221> MOD_RES
   <222> (30)..(30)
   <223> An amino group of a side chain is labeled. A carboxyl group of a C-terminus is amidated.
<400> 7
   Ser Lys Gln Met Glu Glu Glu Ala Val Arg Leu Phe Ile Glu Trp Leu
   1               5                   10                  15
   Lys Asn Gly Gly Pro Ser Ser Gly Ala Pro Pro Pro Ser Lys
               20                  25                  30
<210> 8
   <211> 29
   <212> PRT
   <213> Artificial Sequence
<220>
   <223> An imaging probe for pancreatic islets
<220>
   <221> MOD_RES
   <222> (1)..(1)
   <223> An alpha-amino group of an N-terminus is not modified or is modified by a modified group.
<220>
   <221> MOD_RES
   <222> (29)..(29)
   <223> An amino group of a side chain is labeled. A carboxyl group of a C-terminus is amidated.
<400> 8
   Lys Gln Met Glu Glu Glu Ala Val Arg Leu Phe Ile Glu Trp Leu Lys
   1               5                   10                  15
   Asn Gly Gly Pro Ser Ser Gly Ala Pro Pro Pro Ser Lys
               20                  25
<210> 9
   <211> 32
   <212> PRT
   <213> Artificial Sequence
<220>
   <223> A polypeptide for preparation of precursor of imaging probe for pancreatic islets
<220>
   <221> MOD_RES
   <222> (1)..(1)
   <223> An alpha-amino group of an N-terminus is protected by a Fmoc. A functional group of a side chain is protected by a OBu.
<220>
   <221> MOD_RES
   <222> (3)..(3)
   <223> A functional group of a side chain is protected by a OBu.
<220>
   <221> MOD_RES
   <222> (4)..(4)
   <223> An amino group of a side chain is protected by a Mmt.
<220>
   <221> MOD_RES
   <222> (5)..(5)
   <223> A functional group of a side chain is protected by a Trt.
<220>
   <221> MOD_RES
   <222> (7)..(9)
   <223> A functional group of a side chain is protected by a OBu.
<220>
   <221> MOD_RES
   <222> (12)..(12)
   <223> A functional group of a side chain is protected by a Pdf.
<220>
   <221> MOD_RES
   <222> (16)..(16)
   <223> A functional group of a side chain is protected by a OBu.
<220>
   <221> MOD_RES
   <222> (17)..(17)
   <223> A functional group of a side chain is protected by a Boc.
<220>
   <221> MOD_RES
   <222> (19)..(19)
   <223> An amino group of a side chain is protected by a Mmt.
<220>
   <221> MOD_RES
   <222> (20)..(20)
   <223> A functional group of a side chain is protected by a Trt.
<220>
   <221> MOD_RES
   <222> (24)..(25)
   <223> A functional group of a side chain is protected by a OBu.
<220>
   <221> MOD_RES
   <222> (31)..(31)
   <223> A functional group of a side chain is protected by a OBu.
<220>
   <221> MOD_RES
   <222> (32)..(32)
   <223> An amino group of a side chain is protected by a Boc.
<400> 9
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser Lys
   20 25 30
<210> 10
   <211> 32
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a Fmoc
<220>
   <221> MOD_RES
   <222> (4)..(4)
   <223> An amino group of a side chain is protected by a Fmoc.
<220>
   <221> MOD_RES
   <222> (19)..(19)
   <223> An amino group of a side chain is protected by a Fmoc.
<220>
   <221> MOD_RES
   <222> (32)..(32)
   <223> A carboxyl group of a C-terminus is amidated.
<400> 10
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser Lys
   20 25 30
<210> 11
   <211> 32
   <212> PRT
   <213> Artificial Sequence
<220>
   <223> An imaging probe for pancreatic islets
<220>
   <221> MOD_RES
   <222> (32)..(32)
   <223> An amino group of a side chain is labeled by [18F]fluorobenzoyl. A carboxyl group of a C-terminus is amidated.
<400> 11
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 12
   <211> 31
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a Fmoc.
<220>
   <221> MOD_RES
   <222> (19)..(19)
   <223> An amino group of a side chain is protected by a Fmoc.
<220>
   <221> MOD_RES
   <222> (31)..(31)
   <223> A carboxyl group of a C-terminus is amidated.
<400> 12
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 13
   <211> 31
   <212> PRT
   <213> Artificial Sequence
<220>
   <223> An imaging probe for pancreatic islets
<220>
   <221> MOD_RES
   <222> (4)..(4)
   <223> An amino group of a side chain is labeled by [18F]fluorobenzoyl.
<220>
   <221> MOD_RES
   <222> (31)..(31)
   <223> A carboxyl group of a C-terminus is amidated.
<400> 13
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 14
   <211> 31
   <212> PRT
   <213> Artificial Sequence
<220>
   <223> A precursor of imaging probe for pancreatic islets
<220>
   <221> MOD_RES
   <222> (1)..(1)
   <223> An amino group of an N-terminus is protected by a Fmoc.
<220>
   <221> MOD_RES
   <222> (4)..(4)
   <223> An amino group of a side chain is protected by a Fmoc.
<220>
   <221> MOD_RES
   <222> (31)..(31)
   <223> A carboxyl group of a C-terminus is amidated.
<400> 14
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 15
   <211> 31
   <212> PRT
   <213> Artificial Sequence
<220>
   <223> An imaging probe for pancreatic islets
<220>
   <221> MOD_RES
   <222> (19)..(19)
   <223> An amino group of a side chain is labeled by [18F]fluorobenzoyl.
<220>
   <221> MOD_RES
   <222> (31)..(31)
   <223> A carboxyl group of a C-terminus is amidated.
<400> 15
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 16
   <211> 32
   <212> PRT
   <213> Artificial Sequence
<220>
   <223> A precursor of imaging probe for pancreatic islets
<220>
   <221> MOD_RES
   <222> (1)..(1)
   <223> An alpha-amino group of an N-terminus is modified by an acetyl group.
<220>
   <221> MOD_RES
   <222> (4)..(4)
   <223> An amino group of a side chain is protected by a Fmoc.
<220>
   <221> MOD_RES
   <222> (19)..(19)
   <223> An amino group of a side chain is protected by a Fmoc.
<220>
   <221> MOD_RES
   <222> (32)..(32)
   <223> A carboxyl group of a C-terminus is amidated.
<400> 16
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 17
   <211> 32
   <212> PRT
   <213> Artificial Sequence
<220>
   <223> An imaging probe for pancreatic islets
<220>
   <221> MOD_RES
   <222> (1)..(1)
   <223> An alpha-amino group of an N-terminus is modified by an acetyl group.
<220>
   <221> MOD_RES
   <222> (32)..(32)
   <223> An amino group of a side chain is labeled by [18F]fluorobenzoyl. A carboxyl group of a C-terminus is amidated.
<400> 17
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 18
   <211> 32
   <212> PRT
   <213> Artificial Sequence
<220>
   <223> A probe for use in Binding Assay
<220>
   <221> MOD_RES
   <222> (32)..(32)
   <223> An amino group of a side chain is labeled by [127I]iodobenzoyl. A carboxyl group of a C-terminus is amidated.
<400> 18
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Lys
               20                  25                  30
<210> 19
   <211> 32
   <212> PRT
   <213> Artificial Sequence
<220>
   <223> An imaging probe for pancreatic islets
<220>
   <221> MOD_RES
   <222> (32)..(32)
   <223> An amino group of a side chain is labeled by [1251]iodobenzoyl. A carboxyl group of a C-terminus is amidated.
<400> 19
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser Lys
   20 25 30
<210> 20
   <211> 32
   <212> PRT
   <213> Artificial Sequence
<220>
   <223> An imaging probe for pancreatic islets
<220>
   <221> MOD_RES
   <222> (32)..(32)
   <223> An amino group of a side chain is labeled by [1231]iodobenzoyl. A carboxyl group of a C-terminus is amidated.
<400> 20
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser Lys
   20 25 30